# FASCICULE DE BREVET EUROPEEN

(11) **EP 2 513 143 B1**
(45) Date de publication et mention de la délivrance du brevet: **12.10.2016**
(21) Numéro de dépôt: 10812912.3
(22) Date de dépôt: 16.12.2010
(51) Int. Cl.: C07K 16/30, A61K 39/00, C07K 16/28

(54) **ANTICORPS DIRIGES CONTRE LE RECEPTEUR DE LA TRANSFERRINE ET LEURS UTILISATIONS POUR L'IMMUNOTHERAPIE DES TUMEURS QUI DEPENDENT DU FER**
GEGEN DEN TRANSFERRINREZEPTOR GERICHTETE ANTIKÖRPER UND IHRE VERWENDUNG FÜR DIE IMMUNTHERAPIE VON EISENABHÄNGIGEN TUMOREN
ANTIBODIES DIRECTED AGAINST THE TRANSFERRIN RECEPTOR AND USES THEREOF FOR IMMUNOTHERAPY OF IRON-DEPENDENT TUMOURS

(30) Priorité: 16.12.2009 FR 0906090
(43) Date de publication de la demande: 24.10.2012
(73) Titulaire: Centre National de la Recherche Scientifique (CNRS), 75794 Paris Cedex 16 (FR); The Regents of the University of California, Oakland, CA 94607 (US); Ecole Normale Superieure De Cachan, 94230 Cachan (FR); UNIVERSITE PARIS-SUD (PARIS XI), 91405 Orsay Cédex (FR)
(72) Inventeur: POUL, Marie-Alix, F-34090 Montpellier (FR); CREPIN, Ronan, Philippe, F-78530 Buc (FR); MARKS, James, D, Oakland, CA 94607 (US)
(74) Mandataire: Cabinet Armengaud Aîné
(86) Numéro de dépôt international: PCT/IB2010/055882
(87) Numéro de publication internationale: WO 2011/073943

(56) Documents cités:
- POUL M-A ET AL: "SELECTION OF TUMOR-SPECIFIC INTERNALIZING HUMAN ANTIBODIES FROM PHAGE LIBRARIES", JOURNAL OF MOLECULAR BIOLOGY, LONDON, GB LNKD- DOI:10.1006/JMBI.2000.4026, vol. 301, 1 janvier 2000 (2000-01-01), pages 1149-1161, XP002944684, ISSN: 0022-2836
- CREPIN RONAN ET AL: "Development of Human Single-Chain Antibodies to the Transferrin Receptor that Effectively Antagonize the Growth of Leukemias and Lymphomas", CANCER RESEARCH, vol. 70, no. 13, juillet 2010 (2010-07), pages 5497-5506, XP002599841, ISSN: 0008-5472
- TAKAHASHI S ET AL: "An epitope on the transferrin receptor preferentially exposed during tumor progression in human lymphoma is close to the ligand binding site", BLOOD, AMERICAN SOCIETY OF HEMATOLOGY, US, vol. 77, no. 4, 15 February 1991 (1991-02-15), pages 826-832, XP002594434, ISSN: 0006-4971
- OHNO ET AL: "Simultaneous induction of apoptotic, autophagic, and necrosis-like cell death by monoclonal antibodies recognizing chicken transferrin receptor", BIOCHEMICAL AND BIOPHYSICAL RESEARCH COMMUNICATIONS, ACADEMIC PRESS INC. ORLANDO, FL, US, vol. 367, no. 4, 15 January 2008 (2008-01-15), pages 775-781, XP022449872, ISSN: 0006-291X, DOI: 10.1016/J.BBRC.2008.01.030
- MARIE-ALIX POUL ET AL: "Selection of tumor-specific internalizing human antibodies from phage libraries", JOURNAL OF MOLECULAR BIOLOGY, ACADEMIC PRESS, UNITED KINGDOM, vol. 301, no. 5, 1 January 2000 (2000-01-01), pages 1149-1161, XP008153825, ISSN: 0022-2836, DOI: 10.1006/JMBI.2000.4026
- LEPELLETIER YVES ET AL: "Prevention of mantle lymphoma tumor establishment by routing transferrin receptor toward lysosomal compartments", CANCER RESEARCH, vol. 67, no. 3, February 2007 (2007-02), pages 1145-1154, ISSN: 0008-5472

### SEQUENCE LISTING

<110> CENTRE NATIONAL DE LA RECHERCHE SCIENTIFIQUE (CNRS) The Regents of the University of California
<120> ANTICORPS DIRIGES CONTRE LE RECEPTEUR DE LA TRANSFERRINE ET LEURS UTILISATIONS POUR L'IMMUNOTHERAPIE DES TUMEURS QUI DEPENDENT DU FER
<130> 63062-3620
<150> FR 09/06090
   <151> 2009-12-16
<160> 110
<170> PatentIn version 3.5
<210> 1
   <211> 5
   <212> PRT
   <213> Artificial sequence
<220>
   <223> Acid amino
<400> 1
   Thr Tyr Thr Met His
   1               5
<210> 2
   <211> 17
   <212> PRT
   <213> Artificial sequence
<220>
   <223> Acid amino
<400> 2
   Asp Ile Ala Tyr Asp Gly Ser Thr Lys Tyr Tyr Ala Asp Ser Val Lys
   1               5                   10                  15
   Gly
<210> 3
   <211> 11
   <212> PRT
   <213> Artificial sequence
<220>
   <223> Acid amino
<400> 3
   Asp Ala Val Ala Gly Glu Gly Tyr Phe Asp Leu
   1               5                   10
<210> 4
   <211> 11
   <212> PRT
   <213> Artificial sequence
<220>
   <223> Acid amino
<400> 4
   Gln Gly Asp Ser Leu Arg Ser Tyr Tyr Ala Ser
   1               5                   10
<210> 5
   <211> 7
   <212> PRT
   <213> Artificial sequence
<220>
   <223> Acid amino
<400> 5
   Arg Asn Asn Gln Arg Pro Ser
   1               5
<210> 6
   <211> 11
   <212> PRT
   <213> Artificial sequence
<220>
   <223> Acid amino
<400> 6
   Ala Ala Trp Asp Asp Ser Leu Ser Ala Trp Val
   1               5                   10
<210> 7
   <211> 5
   <212> PRT
   <213> Artificial sequence
<220>
   <223> Acid amino
<400> 7
   Ala Ser Gly Met His
   1 5
<210> 8
   <211> 17
   <212> PRT
   <213> Artificial sequence
<220>
   <223> Acid amino
<400> 8
   Phe Ile Ala Tyr Asp Gly Asn Gln Lys Phe Tyr Ala Asp Ser Val Lys
   1 5 10 15
   Gly
<210> 9
   <211> 10
   <212> PRT
   <213> Artificial sequence
<220>
   <223> Acid amino
<400> 9
   Glu Met Gln Arg Glu Gly Tyr Phe Asp Tyr
   1 5 10
<210> 10
   <211> 11
   <212> PRT
   <213> Artificial sequence
<220>
   <223> Acid amino
<400> 10
   Gln Gly Asp Ser Leu Arg Ser Tyr Tyr Ala Ser
   1 5 10
<210> 11
   <211> 7
   <212> PRT
   <213> Artificial sequence
<220>
   <223> Acid amino
<400> 11
   Gly Lys Asn Asn Arg Pro Ser
   1 5
<210> 12
   <211> 11
   <212> PRT
   <213> Artificial sequence
<220>
   <223> Acid amino
<400> 12
   Ala Thr Trp Asp Asp Asn Leu Ser Gly Pro Ile
   1               5                   10
<210> 13
   <211> 5
   <212> PRT
   <213> Artificial sequence
<220>
   <223> Acid amino
<400> 13
   Asp Tyr Tyr Met Ser
   1               5
<210> 14
   <211> 17
   <212> PRT
   <213> Artificial sequence
<220>
   <223> Acid amino
<400> 14
   Tyr Ile Ser Thr Ser Gly Ser Ser Ile Tyr Tyr Val Asp Ser Val Lys
   1               5                   10                  15
   Gly
<210> 15
   <211> 10
   <212> PRT
   <213> Artificial sequence
<220>
   <223> Acid amino
<400> 15
   Asp Leu His Gly Asp Tyr Ala Phe Asp Ser
   1               5                   10
<210> 16
   <211> 11
   <212> PRT
   <213> Artificial sequence
<220>
   <223> Acid amino
<400> 16
   Gln Gly Asp Ser Leu Arg Ser Tyr Tyr Ala Ser
   1 5 10
<210> 17
   <211> 7
   <212> PRT
   <213> Artificial sequence
<220>
   <223> Acid amino
<400> 17
   Gly Lys Asn Asn Arg Pro Ser
   1 5
<210> 18
   <211> 11
   <212> PRT
   <213> Artificial sequence
<220>
   <223> Acid amino
<400> 18
   Ala Thr Trp Asp Asp Asn Leu Ser Gly Pro Ile
   1 5 10
<210> 19
   <211> 5
   <212> PRT
   <213> Artificial sequence
<220>
   <223> Acid amino
<400> 19
   Ser Tyr Ala Met Ser
   1 5
<210> 20
   <211> 17
   <212> PRT
   <213> Artificial sequence
<220>
   <223> Acid amino
<400> 20
   Ala Ile Ser Gly Ser Gly Gly Ser Thr Tyr Tyr Ala Asp Ser Val Lys
   1               5                   10                  15
   Gly
<210> 21
   <211> 10
   <212> PRT
   <213> Artificial sequence
<220>
   <223> Acid amino
<400> 21
   Val Ser Ser Ser Trp Ser His Phe Asp Tyr
   1               5                   10
<210> 22
   <211> 11
   <212> PRT
   <213> Artificial sequence
<220>
   <223> Acid amino
<400> 22
   Arg Ala Ser Gln Tyr Ile Ser Asn Trp Leu Ala
   1               5                   10
<210> 23
   <211> 7
   <212> PRT
   <213> Artificial sequence
<220>
   <223> Acid amino
<400> 23
   Lys Ala Ser Ser Leu Glu Ser
   1               5
<210> 24
   <211> 10
   <212> PRT
   <213> Artificial sequence
<220>
   <223> Acid amino
<400> 24
   Gln Glu Ser Tyr Asn Thr Pro Leu Phe Thr
   1 5 10
<210> 25
   <211> 5
   <212> PRT
   <213> Artificial sequence
<220>
   <223> Acid amino
<400> 25
   Asn Tyr Ala Ile Asn
   1 5
<210> 26
   <211> 17
   <212> PRT
   <213> Artificial sequence
<220>
   <223> Acid amino
<400> 26
   Asn Ile His His Asp Gly Asn Gly Lys Tyr Tyr Val Asp Ser Val Glu
   1 5 10 15
   Gly
<210> 27
   <211> 9
   <212> PRT
   <213> Artificial sequence
<220>
   <223> Acid amino
<400> 27
   Asp Gly Tyr Gly Gly Tyr Leu Asp Leu
   1 5
<210> 28
   <211> 11
   <212> PRT
   <213> Artificial seqeunce
<220>
   <223> Acid amino
<400> 28
   Gln Gly Asp Ser Leu Arg Ser Tyr Tyr Ala Ser
   1 5 10
<210> 29
   <211> 7
   <212> PRT
   <213> Artificial sequence
<220>
   <223> Acid amino
<400> 29
   Gly Lys Asn Asn Arg Pro Ser
   1               5
<210> 30
   <211> 11
   <212> PRT
   <213> Artificial sequence
<220>
   <223> Acid amino
<400> 30
   Ala Ala Trp Asp Asp Ser Leu Ser Gly Pro Val
   1               5                   10
<210> 31
   <211> 5
   <212> PRT
   <213> Artificial sequence
<220>
   <223> Acid amino
<400> 31
   Ser Tyr Ala Met His
   1               5
<210> 32
   <211> 17
   <212> PRT
   <213> Artificial sequence
<220>
   <223> Acid amino
<400> 32
   Val Ile Ser Tyr Asp Gly Ser Asn Lys Tyr Tyr Ala Asp Ser Val Lys
   1               5                   10                  15
   Gly
<210> 33
   <211> 11
   <212> PRT
   <213> Artificial sequence
<220>
   <223> Acid amino
<400> 33
   Asp Leu Ser Gly Tyr Gly Asp Tyr Pro Asp Tyr
   1               5                   10
<210> 34
   <211> 11
   <212> PRT
   <213> Artificial sequence
<220>
   <223> Acid amino
<400> 34
   Gln Gly Asp Ser Leu Arg Ser Tyr Tyr Ala Ser
   1               5                   10
<210> 35
   <211> 7
   <212> PRT
   <213> Artificial sequence
<220>
   <223> Acid amino
<400> 35
   Gly Arg Asn Glu Arg Pro Ser
   1               5
<210> 36
   <211> 11
   <212> PRT
   <213> Artificial sequence
<220>
   <223> Acid amino
<400> 36
   Ala Gly Trp Asp Asp Ser Leu Thr Gly Pro Val
   1               5                   10
<210> 37
   <211> 119
   <212> PRT
   <213> Artificial sequence
<220>
   <223> Acid amino
<400> 37
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Ser Phe Asn Thr Tyr
               20                  25                  30
   Thr Met His Trp Val Arg Gln Ala Pro Gly Lys Gly Leu Glu Trp Val
           35                  40                  45
   Ala Asp Ile Ala Tyr Asp Gly Ser Thr Lys Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Ala Val Ala Gly Glu Gly Tyr Phe Asp Leu Trp Gly Arg
               100                 105                 110
   Gly Thr Leu Val Thr Val Ser
           115
<210> 38
   <211> 111
   <212> PRT
   <213> Artificial sequence
<220>
   <223> Acid amino
<400> 38
   Ser Gln Ser Ala Leu Thr Gln Asp Pro Ala Val Ser Val Ala Leu Gly
   1 5 10 15
   Gln Thr Val Arg Ile Thr Cys Gln Gly Asp Ser Leu Arg Ser Tyr Tyr
   20 25 30
   Ala Ser Trp Tyr Gln Gln Leu Pro Gly Thr Ala Pro Lys Leu Leu Ile
   35 40 45
   Tyr Arg Asn Asn Gln Arg Pro Ser Gly Val Pro Asp Arg Phe Ser Gly
   50 55 60
   Ser Lys Ser Gly Thr Ser Ala Ser Leu Ala Ile Ser Gly Leu Arg Ser
   65 70 75 80
   Glu Asp Glu Ala Asp Tyr Tyr Cys Ala Ala Trp Asp Asp Ser Leu Ser
   85 90 95
   Ala Trp Val Phe Gly Gly Gly Thr Lys Leu Thr Val Leu Gly Ala
   100 105 110
<210> 39
   <211> 118
   <212> PRT
   <213> Artificial sequence
<220>
   <223> Acid amino
<400> 39
   Gln Val Gln Leu Gln Gln Ser Gly Gly Gly Val Val Gln Pro Gly Gly
   1 5 10 15
   Ser Leu Arg Leu Ser Cys Ala Ala Ser Glu Phe Thr Phe Ser Ala Ser
   20 25 30
   Gly Met His Trp Val Arg Gln Ala Pro Gly Lys Gly Leu Glu Trp Met
   35 40 45
   Ala Phe Ile Ala Tyr Asp Gly Asn Gln Lys Phe Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asp Ser Leu Arg Gly Glu Asp Thr Ala Val Tyr Tyr Cys
   85 90 95
   Ala Lys Glu Met Gln Arg Glu Gly Tyr Phe Asp Tyr Trp Gly Gln Gly
   100 105 110
   Thr Leu Val Thr Val Ser
   115
<210> 40
   <211> 110
   <212> PRT
   <213> Artificial sequence
<220>
   <223> Acid amino
<400> 40
   Ser Asn Phe Met Leu Thr Gln Asp Pro Ala Val Ser Val Ala Leu Gly
   1 5 10 15
   Gln Thr Val Arg Ile Thr Cys Gln Gly Asp Ser Leu Arg Ser Tyr Tyr
   20 25 30
   Ala Ser Trp Tyr Gln Gln Lys Pro Gly Gln Ala Pro Val Leu Val Ile
   35 40 45
   Tyr Gly Lys Asn Asn Arg Pro Ser Gly Ile Pro Asp Arg Phe Ser Gly
       50                  55                  60
   Ser Lys Ser Gly Asn Ser Ala Ser Leu Asp Ile Ser Gly Leu Gln Ser
   65                  70                  75                  80
   Glu Asp Glu Ala Asp Tyr Tyr Cys Ala Thr Trp Asp Asp Asn Leu Ser
                   85                  90                  95
   Gly Pro Ile Phe Gly Gly Gly Thr Lys Val Thr Val Leu Gly
               100                 105                 110
<210> 41
   <211> 118
   <212> PRT
   <213> Artificial sequence
<220>
   <223> Acid amino
<400> 41
   Gln Val Gln Leu Ala Glu Ser Gly Gly Gly Leu Val Lys Pro Gly Gly
   1               5                   10                  15
   Ser Leu Arg Leu Ser Cys Ala Ala Ser Gly Phe Thr Phe Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Ser Thr Ser Gly Ser Ser Ile Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asp Ser Leu Arg Asp Asp Asp Thr Ala Val Tyr Tyr Cys
                   85                  90                  95
   Ala Arg Asp Leu His Gly Asp Tyr Ala Phe Asp Ser Trp Gly Gln Gly
               100                 105                 110
   Thr Leu Val Thr Val Ser
           115
<210> 42
   <211> 109
   <212> PRT
   <213> Artificial sequence
<220>
   <223> Acid amino
<400> 42
   Ser Ser Glu Leu Thr Gln Asp Pro Ala Val Ser Val Ala Leu Gly Gln
   1 5 10 15
   Thr Val Arg Ile Thr Cys Gln Gly Asp Ser Leu Arg Ser Tyr Tyr Ala
   20 25 30
   Ser Trp Tyr Gln Gln Lys Pro Gly Gln Ala Pro Val Leu Val Ile Tyr
   35 40 45
   Gly Lys Asn Asn Arg Pro Ser Gly Ile Pro Asp Arg Phe Ser Gly Ser
   50 55 60
   Lys Ser Gly Asn Ser Ala Ser Leu Asp Ile Ser Gly Leu Gln Ser Glu
   65 70 75 80
   Asp Glu Ala Asp Tyr Tyr Cys Ala Thr Trp Asp Asp Asn Leu Ser Gly
   85 90 95
   Pro Ile Phe Gly Gly Gly Thr Lys Val Thr Val Leu Gly
   100 105
<210> 43
   <211> 118
   <212> PRT
   <213> Artificial sequence
<220>
   <223> Acid amino
<400> 43
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Ser Ser Trp Ser His Phe Asp Tyr Trp Gly Gln Gly
               100                 105                 110
   Thr Leu Val Thr Val Ser
           115
<210> 44
   <211> 110
   <212> PRT
   <213> Artificial sequence
<220>
   <223> Acid amino
<400> 44
   Ser Asp Val Val Met Thr Gln Ser Pro Ser Thr Leu Ser Ala Ser Val
   1               5                   10                  15
   Gly Asp Arg Val Thr Ile Thr Cys Arg Ala Ser Gln Tyr Ile Ser Asn
               20                  25                  30
   Trp Leu Ala Trp Tyr Gln Gln Lys Pro Gly Lys Ala Pro Lys Leu Leu
           35                  40                  45
   Ile Tyr Lys Ala Ser Ser Leu Glu Ser Gly Val Pro Ser Arg Phe Ser
       50                  55                  60
   Gly Ser Gly Ser Gly Thr Glu Phe Thr Leu Thr Ile Ser Ser Leu Gln
   65                  70                  75                  80
   Pro Glu Asp Phe Ala Thr Tyr Tyr Cys Gln Glu Ser Tyr Asn Thr Pro
                   85                  90                  95
   Leu Phe Thr Phe Gly Pro Gly Thr Lys Leu Glu Ile Lys Arg
               100                 105                 110
<210> 45
   <211> 117
   <212> PRT
   <213> Artificial sequence
<220>
   <223> Acid amino
<400> 45
   Gln Val Gln Leu Val Glu Ser Gly Gly Gly Leu Val Glu Pro Gly Gly
   1               5                   10                  15
   Ser Leu Arg Leu Ser Cys Ala Ala Ser Gly Phe Thr Phe Ser Asn Tyr
               20                  25                  30
   Ala Ile Asn Trp Val Arg Gln Ala Pro Gly Lys Gly Leu Glu Trp Val
           35                  40                  45
   Ala Asn Ile His His Asp Gly Asn Gly Lys Tyr Tyr Val Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asp Ser Leu Arg Ala Glu Asp Thr Ala Ile Tyr Tyr Cys
                   85                  90                  95
   Ala Arg Asp Gly Tyr Gly Gly Tyr Leu Asp Leu Trp Gly Gln Gly Thr
               100                 105                 110
   Leu Val Thr Val Ser
           115
<210> 46
   <211> 109
   <212> PRT
   <213> Artificial sequence
<220>
   <223> Acid amino
<400> 46
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Ile Tyr
           35                  40                  45
   Gly Lys Asn Asn Arg Pro Ser Gly Ile Pro Asp Arg Phe Ser Gly Ser
       50                  55                  60
   Gly Ser Gly Asn Thr Ala Ser Leu Thr Ile Thr Gly Ala Gln Ala Glu
   65                  70                  75                  80
   Asp Glu Ala Asp Tyr Tyr Cys Ala Ala Trp Asp Asp Ser Leu Ser Gly
                   85                  90                  95
   Pro Val Phe Gly Gly Gly Thr Lys Val Thr Val Leu Gly
               100                 105
<210> 47
   <211> 119
   <212> PRT
   <213> Artificial sequence
<220>
   <223> Acid amino
<400> 47
   Gln Val Gln Leu Gln Glu Ser Gly Gly Gly Val Val Gln Pro Gly Arg
   1               5                   10                  15
   Ser Leu Arg Leu Ser Cys Ala Ala Ser Arg Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Ser Gly Tyr Gly Asp Tyr Pro Asp Tyr Trp Gly Gln
               100                 105                 110
   Gly Thr Leu Val Thr Val Ser
           115
<210> 48
   <211> 109
   <212> PRT
   <213> Artificial sequence
<220>
   <223> Acid amino
<400> 48
   Ser Ser Glu Leu Thr Gln Asp Pro Ala Val Ser Val Ala Leu Gly Gln
   1               5                   10                  15
   Thr Val Arg Ile Thr Cys Gln Gly Asp Ser Leu Arg Ser Tyr Tyr Ala
               20                  25                  30
   Ser Trp Tyr Gln Gln Lys Pro Gly Gln Ala Pro Val Leu Val Met Tyr
           35                  40                  45
   Gly Arg Asn Glu Arg Pro Ser Gly Val Pro Asp Arg Phe Ser Gly Ser
       50                  55                  60
   Lys Ser Gly Thr Ser Ala Ser Leu Ala Ile Ser Gly Leu Gln Pro Glu
   65                  70                  75                  80
   Asp Glu Ala Asn Tyr Tyr Cys Ala Gly Trp Asp Asp Ser Leu Thr Gly
                   85                  90                  95
   Pro Val Phe Gly Gly Gly Thr Lys Leu Thr Val Leu Gly
               100                 105
<210> 49
   <211> 245
   <212> PRT
   <213> Artificial sequence
<220>
   <223> Acid amino
<400> 49
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Ser Phe Asn Thr Tyr
               20                  25                  30
   Thr Met His Trp Val Arg Gln Ala Pro Gly Lys Gly Leu Glu Trp Val
           35                  40                  45
   Ala Asp Ile Ala Tyr Asp Gly Ser Thr Lys Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Ala Val Ala Gly Glu Gly Tyr Phe Asp Leu Trp Gly Arg
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Gln Ser Ala Leu Thr Gln Asp Pro Ala
       130                 135                 140
   Val Ser Val Ala Leu Gly Gln Thr Val Arg Ile Thr Cys Gln Gly Asp
   145                 150                 155                 160
   Ser Leu Arg Ser Tyr Tyr Ala Ser Trp Tyr Gln Gln Leu Pro Gly Thr
                   165                 170                 175
   Ala Pro Lys Leu Leu Ile Tyr Arg Asn Asn Gln Arg Pro Ser Gly Val
               180                 185                 190
   Pro Asp Arg Phe Ser Gly Ser Lys Ser Gly Thr Ser Ala Ser Leu Ala
           195                 200                 205
   Ile Ser Gly Leu Arg Ser Glu Asp Glu Ala Asp Tyr Tyr Cys Ala Ala
       210                 215                 220
   Trp Asp Asp Ser Leu Ser Ala Trp Val Phe Gly Gly Gly Thr Lys Leu
   225                 230                 235                 240
   Thr Val Leu Gly Ala
                   245
<210> 50
   <211> 243
   <212> PRT
   <213> Artificial sequence
<220>
   <223> Acid amino
<400> 50
   Gln Val Gln Leu Gln Gln Ser Gly Gly Gly Val Val Gln Pro Gly Gly
   1               5                   10                  15
   Ser Leu Arg Leu Ser Cys Ala Ala Ser Glu Phe Thr Phe Ser Ala Ser
               20                  25                  30
   Gly Met His Trp Val Arg Gln Ala Pro Gly Lys Gly Leu Glu Trp Met
           35                  40                  45
   Ala Phe Ile Ala Tyr Asp Gly Asn Gln Lys Phe Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asp Ser Leu Arg Gly Glu Asp Thr Ala Val Tyr Tyr Cys
                   85                  90                  95
   Ala Lys Glu Met Gln Arg Glu Gly Tyr Phe Asp Tyr Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Gly Ser Asn Phe Met Leu Thr Gln Asp Pro Ala Val
       130                 135                 140
   Ser Val Ala Leu Gly Gln Thr Val Arg Ile Thr Cys Gln Gly Asp Ser
   145                 150                 155                 160
   Leu Arg Ser Tyr Tyr Ala Ser Trp Tyr Gln Gln Lys Pro Gly Gln Ala
                   165                 170                 175
   Pro Val Leu Val Ile Tyr Gly Lys Asn Asn Arg Pro Ser Gly Ile Pro
               180                 185                 190
   Asp Arg Phe Ser Gly Ser Lys Ser Gly Asn Ser Ala Ser Leu Asp Ile
           195                 200                 205
   Ser Gly Leu Gln Ser Glu Asp Glu Ala Asp Tyr Tyr Cys Ala Thr Trp
       210                 215                 220
   Asp Asp Asn Leu Ser Gly Pro Ile Phe Gly Gly Gly Thr Lys Val Thr
   225                 230                 235                 240
   Val Leu Gly
<210> 51
   <211> 242
   <212> PRT
   <213> Artificial sequence
<220>
   <223> Acid amino
<400> 51
   Gln Val Gln Leu Ala Glu Ser Gly Gly Gly Leu Val Lys Pro Gly Gly
   1               5                   10                  15
   Ser Leu Arg Leu Ser Cys Ala Ala Ser Gly Phe Thr Phe Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Ser Thr Ser Gly Ser Ser Ile Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asp Ser Leu Arg Asp Asp Asp Thr Ala Val Tyr Tyr Cys
                   85                  90                  95
   Ala Arg Asp Leu His Gly Asp Tyr Ala Phe Asp Ser Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Gly Ser Ser Glu Leu Thr Gln Asp Pro Ala Val Ser
       130                 135                 140
   Val Ala Leu Gly Gln Thr Val Arg Ile Thr Cys Gln Gly Asp Ser Leu
   145                 150                 155                 160
   Arg Ser Tyr Tyr Ala Ser Trp Tyr Gln Gln Lys Pro Gly Gln Ala Pro
                   165                 170                 175
   Val Leu Val Ile Tyr Gly Lys Asn Asn Arg Pro Ser Gly Ile Pro Asp
               180                 185                 190
   Arg Phe Ser Gly Ser Lys Ser Gly Asn Ser Ala Ser Leu Asp Ile Ser
           195                 200                 205
   Gly Leu Gln Ser Glu Asp Glu Ala Asp Tyr Tyr Cys Ala Thr Trp Asp
       210                 215                 220
   Asp Asn Leu Ser Gly Pro Ile Phe Gly Gly Gly Thr Lys Val Thr Val
   225                 230                 235                 240
   Leu Gly
<210> 52
   <211> 243
   <212> PRT
   <213> Artificial sequence
<220>
   <223> Acid amino
<400> 52
   Gln Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Ser Ser Trp Ser His Phe Asp Tyr Trp Gly Gln Gly
               100                 105                 110
   Thr Leu Val Thr Val Ser Ser Gly Gly Gly Gly Ser Gly Gly Gly Gly
           115                 120                 125
   Ser Gly Gly Gly Gly Ser Asp Val Val Met Thr Gln Ser Pro Ser Thr
       130                 135                 140
   Leu Ser Ala Ser Val Gly Asp Arg Val Thr Ile Thr Cys Arg Ala Ser
   145                 150                 155                 160
   Gln Tyr Ile Ser Asn Trp Leu Ala Trp Tyr Gln Gln Lys Pro Gly Lys
                   165                 170                 175
   Ala Pro Lys Leu Leu Ile Tyr Lys Ala Ser Ser Leu Glu Ser Gly Val
               180                 185                 190
   Pro Ser Arg Phe Ser Gly Ser Gly Ser Gly Thr Glu Phe Thr Leu Thr
           195                 200                 205
   Ile Ser Ser Leu Gln Pro Glu Asp Phe Ala Thr Tyr Tyr Cys Gln Glu
   210 215 220
   Ser Tyr Asn Thr Pro Leu Phe Thr Phe Gly Pro Gly Thr Lys Leu Glu
   225 230 235 240
   Ile Lys Arg
<210> 53
   <211> 241
   <212> PRT
   <213> Artificial sequence
<220>
   <223> Acid amino
<400> 53
   Gln Val Gln Leu Val Glu Ser Gly Gly Gly Leu Val Glu Pro Gly Gly
   1 5 10 15
   Ser Leu Arg Leu Ser Cys Ala Ala Ser Gly Phe Thr Phe Ser Asn Tyr
   20 25 30
   Ala Ile Asn Trp Val Arg Gln Ala Pro Gly Lys Gly Leu Glu Trp Val
   35 40 45
   Ala Asn Ile His His Asp Gly Asn Gly Lys Tyr Tyr Val Asp Ser Val
   50 55 60
   Glu Gly Arg Phe Thr Ile Ser Arg Asp Asn Ala Lys Asn Ser Leu Tyr
   65 70 75 80
   Leu Gln Met Asp Ser Leu Arg Ala Glu Asp Thr Ala Ile Tyr Tyr Cys
   85 90 95
   Ala Arg Asp Gly Tyr Gly Gly Tyr Leu Asp Leu Trp Gly Gln Gly Thr
   100 105 110
   Leu Val Thr Val Ser Ser Gly Gly Gly Gly Ser Gly Gly Gly Gly Ser
   115 120 125
   Gly Gly Gly Gly Ser Ser Glu Leu Thr Gln Asp Pro Ala Val Ser Val
   130 135 140
   Ala Leu Gly Gln Thr Val Arg Ile Thr Cys Gln Gly Asp Ser Leu Arg
   145 150 155 160
   Ser Tyr Tyr Ala Ser Trp Tyr Gln Gln Lys Pro Gly Gln Ala Pro Val
   165 170 175
   Leu Val Ile Tyr Gly Lys Asn Asn Arg Pro Ser Gly Ile Pro Asp Arg
   180 185 190
   Phe Ser Gly Ser Gly Ser Gly Asn Thr Ala Ser Leu Thr Ile Thr Gly
   195 200 205
   Ala Gln Ala Glu Asp Glu Ala Asp Tyr Tyr Cys Ala Ala Trp Asp Asp
   210 215 220
   Ser Leu Ser Gly Pro Val Phe Gly Gly Gly Thr Lys Val Thr Val Leu
   225 230 235 240
   Gly
<210> 54
   <211> 243
   <212> PRT
   <213> Artificial sequence
<220>
   <223> Acid amino
<400> 54
   Gln Val Gln Leu Gln Glu Ser Gly Gly Gly Val Val Gln Pro Gly Arg
   1               5                   10                  15
   Ser Leu Arg Leu Ser Cys Ala Ala Ser Arg Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Ser Gly Tyr Gly Asp Tyr Pro Asp Tyr Trp Gly Gln
               100                 105                 110
   Gly Thr Leu Val Thr Val Ser Ser Gly Gly Gly Gly Ser Gly Gly Gly
           115                 120                 125
   Gly Ser Gly Gly Gly Gly Ser Ser Glu Leu Thr Gln Asp Pro Ala Val
       130                 135                 140
   Ser Val Ala Leu Gly Gln Thr Val Arg Ile Thr Cys Gln Gly Asp Ser
   145                 150                 155                 160
   Leu Arg Ser Tyr Tyr Ala Ser Trp Tyr Gln Gln Lys Pro Gly Gln Ala
                   165                 170                 175
   Pro Val Leu Val Met Tyr Gly Arg Asn Glu Arg Pro Ser Gly Val Pro
               180                 185                 190
   Asp Arg Phe Ser Gly Ser Lys Ser Gly Thr Ser Ala Ser Leu Ala Ile
           195                 200                 205
   Ser Gly Leu Gln Pro Glu Asp Glu Ala Asn Tyr Tyr Cys Ala Gly Trp
       210                 215                 220
   Asp Asp Ser Leu Thr Gly Pro Val Phe Gly Gly Gly Thr Lys Leu Thr
   225                 230                 235                 240
   Val Leu Gly
<210> 55
   <211> 15
   <212> DNA
   <213> Artificial sequence
<220>
   <223> Nucleotidic
<400> 55
   acctatacta tgcac 15
<210> 56
   <211> 51
   <212> DNA
   <213> Artificial sequence
<220>
   <223> Nucleotidic
<400> 56
   gatatagcat atgatgggag tactaaatac tacgcagact ctgtgaaggg c 51
<210> 57
   <211> 33
   <212> DNA
   <213> Artificial sequence
<220>
   <223> Nucleotidic
<400> 57
   gatgcagtgg ctggtgaagg gtacttcgat ctc 33
<210> 58
   <211> 33
   <212> DNA
   <213> Artificial sequence
<220>
   <223> Nucleotidic
<400> 58
   caaggagaca gcctcagaag ctattatgca agt 33
<210> 59
   <211> 21
   <212> DNA
   <213> Artificial sequence
<220>
   <223> Nucleotidic
<400> 59
   aggaataatc agcggccctc a 21
<210> 60
   <211> 33
   <212> DNA
   <213> Artificial sequence
<220>
   <223> Nucleotidic
<400> 60
   gcagcatggg atgacagcct gagtgcctgg gtg 33
<210> 61
   <211> 15
   <212> DNA
   <213> Artificial sequence
<220>
   <223> Nucleotidic
<400> 61
   gcctctggca tgcac 15
<210> 62
   <211> 51
   <212> DNA
   <213> Artificial sequence
<220>
   <223> Nucleotidic
<400> 62
   tttatagcct atgatggaaa tcaaaaattc tatgcagact ccgtgaaggg c 51
<210> 63
   <211> 30
   <212> DNA
   <213> Artificial sequence
<220>
   <223> Nucleotidic
<400> 63
   gaaatgcaac gcgaggggta ctttgactac 30
<210> 64
   <211> 33
   <212> DNA
   <213> Artificial sequence
<220>
   <223> Nucleotidic
<400> 64
   caaggagaca gcctcagaag ctattatgca agc 33
<210> 65
   <211> 21
   <212> DNA
   <213> Artificial sequence
<220>
   <223> Nucleotidic
<400> 65
   ggtaaaaaca accggccctc a 21
<210> 66
   <211> 33
   <212> DNA
   <213> Artificial sequence
<220>
   <223> Nucleotidic
<400> 66
   gcaacatggg atgacaacct gagtggtccg ata 33
<210> 67
   <211> 15
   <212> DNA
   <213> Artificial sequence
<220>
   <223> Nucleotidic
<400> 67
   gactactaca tgagc 15
<210> 68
   <211> 51
   <212> DNA
   <213> Artificial sequence
<220>
   <223> Nucleotidic
<400> 68
   tacattagta ctagtggtag tagcatatac tatgtagact ctgtgaaggg c 51
<210> 69
   <211> 30
   <212> DNA
   <213> Artificial sequence
<220>
   <223> Nucleotidic
<400> 69
   gatcttcacg gtgactatgc ctttgactcc 30
<210> 70
   <211> 33
   <212> DNA
   <213> Artificial sequence
<220>
   <223> Nucleotidic
<400> 70
   caaggagaca gtctcagaag ttattatgca agc 33
<210> 71
   <211> 21
   <212> DNA
   <213> Artificial sequence
<220>
   <223> Nucleotidic
<400> 71 21
   ggtaaaaaca accggccctc a 21
<210> 72
   <211> 33
   <212> DNA
   <213> Artificial sequence
<220>
   <223> Nucleotidic
<400> 72
   gcaacatggg atgacaacct gagtggtccg ata 33
<210> 73
   <211> 15
   <212> DNA
   <213> Artificial sequence
<220>
   <223> Nucleotidic
<400> 73
   agctatgcca tgagc 15
<210> 74
   <211> 51
   <212> DNA
   <213> Artificial sequence
<220>
   <223> Nucleotidic
<400> 74
   gctattagtg gtagtggtgg tagcacatac tacgcagact ccgtgaaggg c 51
<210> 75
   <211> 30
   <212> DNA
   <213> Artificial sequence
<220>
   <223> Nucleotidic
<400> 75
   gtgagcagca gctggtccca ttttgactac 30
<210> 76
   <211> 33
   <212> DNA
   <213> Artificial sequence
<220>
   <223> Nucleotidic
<400> 76
   cgggccagtc agtatattag taactggttg gcc 33
<210> 77
   <211> 21
   <212> DNA
   <213> Artificial sequence
<220>
   <223> Nucleotidic
<400> 77
   aaggcgtcta gtttagaaag t 21
<210> 78
   <211> 30
   <212> DNA
   <213> Artificial sequence
<220>
   <223> Nucleotidic
<400> 78
   caagagagtt acaatacccc cttattcact 30
<210> 79
   <211> 15
   <212> DNA
   <213> Artificial sequence
<220>
   <223> Nucleotidic
<400> 79
   aactatgcca taaac 15
<210> 80
   <211> 51
   <212> DNA
   <213> Artificial sequence
<220>
   <223> Nucleotidic
<400> 80
   aacatacacc acgatggaaa tggtaaatac tatgtggact ctgtggaggg c 51
<210> 81
   <211> 27
   <212> DNA
   <213> Artificial sequence
<220>
   <223> Nucleotidic
<400> 81
   gacggctacg ggggttacct tgacttg 27
<210> 82
   <211> 33
   <212> DNA
   <213> Artificial sequence
<220>
   <223> Nucleotidic
<400> 82
   caaggagaca gcctcagaag ctattatgca agc 33
<210> 83
   <211> 21
   <212> DNA
   <213> Artificial sequence
<220>
   <223> Nucleotidic
<400> 83
   ggtaaaaaca accggccctc a 21
<210> 84
   <211> 33
   <212> DNA
   <213> Artificial sequence
<220>
   <223> Nucleotidic
<400> 84
   gcagcatggg atgacagcct gagtggtccg gtg 33
<210> 85
   <211> 15
   <212> DNA
   <213> Artificial sequence
<220>
   <223> Nucleotidic
<400> 85
   agctatgcta tgcac 15
<210> 86
   <211> 51
   <212> DNA
   <213> Artificial sequence
<220>
   <223> Nucleotidic
<400> 86
   gttatatcat atgatggaag caataaatac tacgcagact ccgtgaaggg c 51
<210> 87
   <211> 33
   <212> DNA
   <213> Artificial sequence
<220>
   <223> Nucleotidic
<400> 87
   gatctctcgg ggtacggtga ctaccctgac tac 33
<210> 88
   <211> 33
   <212> DNA
   <213> Artificial sequence
<220>
   <223> Nucleotidic
<400> 88
   caaggagaca gcctcagaag ctattatgca agc 33
<210> 89
   <211> 21
   <212> DNA
   <213> Artificial sequence
<220>
   <223> Nucleotidic
<400> 89
   ggtagaaacg agcggccctc a 21
<210> 90
   <211> 33
   <212> DNA
   <213> Artificial sequence
<220>
   <223> Nucleotidic
<400> 90
   gcagggtggg atgacagcct gactggtccg gtg 33
<210> 91
   <211> 357
   <212> DNA
   <213> Artificial sequence
<220>
   <223> Nucleotidic
<400> 91
   caggtgcagc tgcaggagtc ggggggaggc ttggtacagc ctggggggtc cctgagactc 60
   tcctgtgcag cctctggatt ctccttcaac acctatacta tgcactgggt ccgccaggct 120
   ccaggcaagg ggctggagtg ggtggcggat atagcatatg atgggagtac taaatactac 180
   gcagactctg tgaagggccg attcaccatc tccagagaca atgccaagaa ctcactgtat 240
   ctgcaaatga acagcctgag agccgaggac acggctgtgt attactgtgc gagagatgca 300
   gtggctggtg aagggtactt cgatctctgg ggccgtggca ccctggtcac cgtctcc 357
<210> 92
   <211> 333
   <212> DNA
   <213> Artificial sequence
<220>
   <223> Nucleotidic
<400> 92
   tcgcagtctg ctctgactca ggaccctgct gtgtctgtgg ccttgggaca gacagtcagg 60
   atcacatgcc aaggagacag cctcagaagc tattatgcaa gttggtacca gcagctccca 120
   ggaacggccc ccaaactcct catctatagg aataatcagc ggccctcagg ggtccctgac 180
   cgattctctg gctccaagtc tggcacctca gcctccctgg ccatcagtgg gctccggtcc 240
   gaggatgagg ctgattatta ctgtgcagca tgggatgaca gcctgagtgc ctgggtgttc 300
   ggcggaggga ccaagctgac cgtcctaggt gcg 333
<210> 93
   <211> 354
   <212> DNA
   <213> Artificial sequence
<220>
   <223> Nucleotidic
<400> 93
   caggtacagc tgcagcagtc agggggaggc gtggtccagc ctggggggtc cctgagactc 60
   tcctgtgcag cgtctgagtt caccttcagt gcctctggca tgcactgggt ccgccaggct 120
   ccaggcaagg gcctggaatg gatggcattt atagcctatg atggaaatca aaaattctat 180
   gcagactccg tgaagggccg attcaccatc tccagagaca attccaagaa cactctgtat 240
   ctgcaaatgg acagcctgag aggtgaggac acggctgtgt attactgtgc gaaagaaatg 300
   caacgcgagg ggtactttga ctactggggc cagggaaccc tggtcaccgt ctcc 354
<210> 94
   <211> 330
   <212> DNA
   <213> Artificial sequence
<220>
   <223> Nucleotidic
<400> 94
   tcgaatttta tgctgactca ggaccctgct gtgtctgtgg ccttgggaca gacagtcagg      60
   atcacatgcc aaggagacag cctcagaagc tattatgcaa gctggtacca gcagaagcca     120
   ggacaggccc ctgtacttgt catctatggt aaaaacaacc ggccctcagg gatcccagac     180
   cgattctctg gctccaagtc tggcaactca gcctccctgg acatcagtgg gctccagtct     240
   gaggatgagg ctgattatta ttgtgcaaca tgggatgaca acctgagtgg tccgatattc     300
   ggcggaggga ccaaggtcac cgtcctaggt                                      330
<210> 95
   <211> 354
   <212> DNA
   <213> Artificial sequence
<220>
   <223> Nucleotidic
<400> 95
   caggtgcagc tggcggagtc tgggggaggc ttggtcaagc ctggagggtc cctgagactc      60
   tcctgtgcag cctctggatt caccttcagt gactactaca tgagctggat ccgccaggct     120
   ccagggaagg ggctggagtg ggtttcatac attagtacta gtggtagtag catatactat     180
   gtagactctg tgaagggccg attcaccatc tccagggaca acgccaagaa ctcactgtat     240
   ctgcaaatgg acagcctgag agacgacgac acggctgttt attactgtgc gagagatctt     300
   cacggtgact atgcctttga ctcctggggc cagggaaccc tggtcaccgt ctcc           354
<210> 96
   <211> 327
   <212> DNA
   <213> Artificial sequence
<220>
   <223> Nucleotidic
<400> 96
   tcgtctgagc tgactcagga ccctgctgtg tctgtggcct tgggacagac agtcaggatc        60
   acatgccaag gagacagtct cagaagttat tatgcaagct ggtaccagca gaagccagga       120
   caggcccctg tacttgtcat ctatggtaaa aacaaccggc cctcagggat cccagaccga       180
   ttctctggct ccaagtctgg caactcagcc tccctggaca tcagtgggct ccagtctgag       240
   gatgaggctg attattattg tgcaacatgg gatgacaacc tgagtggtcc gatattcggc       300
   ggagggacca aggtcaccgt cctaggt                                           327
<210> 97
   <211> 354
   <212> DNA
   <213> Artificial sequence
<220>
   <223> Nucleotidic
<400> 97
   caggtgcagc tgcaggagtc ggggggaggc ttggtacagc ctggggggtc cctgagactc        60
   tcctgtgcag cctctggatt cacctttagc agctatgcca tgagctgggt ccgccaggct       120
   ccagggaagg ggctggagtg ggtctcagct attagtggta gtggtggtag cacatactac       180
   gcagactccg tgaagggccg gttcaccatc tccagagaca attccaagaa cacgctgtat       240
   ctgcaaatga acagcctgag agccgaggac acggccgtat attactgtgc gaaagtgagc       300
   agcagctggt cccattttga ctactggggc cagggaaccc tggtcaccgt ctcc             354
<210> 98
   <211> 330
   <212> DNA
   <213> Artificial sequence
<220>
   <223> Nucleotidic
<400> 98
   tcggatgttg tgatgactca gtctccttcc accctgtctg catctgtagg agacagagtc        60
   accatcactt gccgggccag tcagtatatt agtaactggt tggcctggta tcagcagaaa       120
   ccagggaaag cccctaagct cctgatctat aaggcgtcta gtttagaaag tggggtccca       180
   tcaaggttca gcggcagtgg atctgggaca gagttcactc tcaccatcag cagtctgcaa       240
   cctgaagatt ttgcaactta ctactgtcaa gagagttaca ataccccctt attcactttc      300
   ggccctggga ccaagctgga gatcaaacgt                                        330
<210> 99
   <211> 351
   <212> DNA
   <213> Artificial sequence
<220>
   <223> Nucleotidic
<400> 99
   caggtgcagc tggtggagtc tgggggaggc ttagtggagc ctggggggtc cctgagactc      60
   tcctgtgcgg cctctggatt cacctttagc aactatgcca taaactgggt ccgccaggct     120
   ccagggaagg ggctggagtg ggtggccaac atacaccacg atggaaatgg taaatactat     180
   gtggactctg tggagggccg attcaccatc tccagagaca acgccaagaa ttctctgtat     240
   ctgcaaatgg acagcctgag agccgaggac acggccattt attactgtgc gcgagacggc     300
   tacgggggtt accttgactt gtggggccag ggaaccctgg tcaccgtctc c              351
<210> 100
   <211> 327
   <212> DNA
   <213> Artificial sequence
<220>
   <223> Nucleotidic
<400> 100
   tcgtctgagc tgactcagga ccctgctgtg tctgtggcct tgggacagac agtcaggatc      60
   acatgccaag gagacagcct cagaagctat tatgcaagct ggtaccagca gaagccagga     120
   caggcccctg tacttgtcat ctatggtaaa aacaaccggc cctcagggat cccagaccga     180
   ttctctggct ccggctcagg aaacacagct tccttgacca tcactggggc tcaggcggaa     240
   gatgaggctg actattactg tgcagcatgg gatgacagcc tgagtggtcc ggtgttcggc     300
   ggagggacca aggtcaccgt cctaggt                                          327
<210> 101
   <211> 357
   <212> DNA
   <213> Artificial sequence
<220>
   <223> Nucleotidic
<400> 101
   caggtgcagc tgcaggagtc ggggggaggc gtggtccagc ctgggaggtc cctgagactc       60
   tcctgtgcag cctctcgatt caccttcagt agctatgcta tgcactgggt ccgccaggct     120
   ccaggcaagg ggctggagtg ggtggcagtt atatcatatg atggaagcaa taaatactac     180
   gcagactccg tgaagggccg attcaccatc tccagagaca attccaagaa cacgctgtat     240
   ctgcaaatga acagcctgag agctgaggac acggctgtgt attactgtgc gagagatctc 300
   tcggggtacg gtgactaccc tgactactgg ggccagggaa ccctggtcac cgtctcc 357
<210> 102
   <211> 327
   <212> DNA
   <213> Artificial sequence
<220>
   <223> Nucleotidic
<400> 102
   tcgtctgagc tgactcagga ccctgctgtg tctgtggcct tgggacagac agtcagaatc 60
   acatgccaag gagacagcct cagaagctat tatgcaagct ggtaccagca gaagccagga 120
   caggcccctg tacttgtcat gtatggtaga aacgagcggc cctcaggggt tcctgaccga 180
   ttctctggct ccaagtctgg cacctctgcc tccctggcca tcagtggcct ccagccagag 240
   gatgaggcta attattactg tgcagggtgg gatgacagcc tgactggtcc ggtgttcggc 300
   ggagggacca agctgaccgt cctaggt 327
<210> 103
   <211> 735
   <212> DNA
   <213> Artificial sequence
<220>
   <223> Nucleotidic
<400> 103
   caggtgcagc tgcaggagtc ggggggaggc ttggtacagc ctggggggtc cctgagactc 60
   tcctgtgcag cctctggatt ctccttcaac acctatacta tgcactgggt ccgccaggct 120
   ccaggcaagg ggctggagtg ggtggcggat atagcatatg atgggagtac taaatactac 180
   gcagactctg tgaagggccg attcaccatc tccagagaca atgccaagaa ctcactgtat 240
   ctgcaaatga acagcctgag agccgaggac acggctgtgt attactgtgc gagagatgca 300
   gtggctggtg aagggtactt cgatctctgg ggccgtggca ccctggtcac cgtctcctca 360
   ggtggaggcg gttcaggcgg aggtggctct ggcggtggcg gatcgcagtc tgctctgact 420
   caggaccctg ctgtgtctgt ggccttggga cagacagtca ggatcacatg ccaaggagac 480
   agcctcagaa gctattatgc aagttggtac cagcagctcc caggaacggc ccccaaactc 540
   ctcatctata ggaataatca gcggccctca ggggtccctg accgattctc tggctccaag 600
   tctggcacct cagcctccct ggccatcagt gggctccggt ccgaggatga ggctgattat 660
   tactgtgcag catgggatga cagcctgagt gcctgggtgt tcggcggagg gaccaagctg 720
   accgtcctag gtgcg 735
<210> 104
   <211> 729
   <212> DNA
   <213> Artificial sequence
<220>
   <223> Nucleotidic
<400> 104
   caggtacagc tgcagcagtc agggggaggc gtggtccagc ctggggggtc cctgagactc 60
   tcctgtgcag cgtctgagtt caccttcagt gcctctggca tgcactgggt ccgccaggct 120
   ccaggcaagg gcctggaatg gatggcattt atagcctatg atggaaatca aaaattctat 180
   gcagactccg tgaagggccg attcaccatc tccagagaca attccaagaa cactctgtat 240
   ctgcaaatgg acagcctgag aggtgaggac acggctgtgt attactgtgc gaaagaaatg 300
   caacgcgagg ggtactttga ctactggggc cagggaaccc tggtcaccgt ctcctcaggt 360
   ggaggcggtt caggcggagg tggctctggc ggtggcggat cgaattttat gctgactcag 420
   gaccctgctg tgtctgtggc cttgggacag acagtcagga tcacatgcca aggagacagc 480
   ctcagaagct attatgcaag ctggtaccag cagaagccag gacaggcccc tgtacttgtc 540
   atctatggta aaaacaaccg gccctcaggg atcccagacc gattctctgg ctccaagtct 600
   ggcaactcag cctccctgga catcagtggg ctccagtctg aggatgaggc tgattattat 660
   tgtgcaacat gggatgacaa cctgagtggt ccgatattcg gcggagggac caaggtcacc 720
   gtcctaggt 729
<210> 105
   <211> 726
   <212> DNA
   <213> Artificial sequence
<220>
   <223> Nucleotidic
<400> 105
   caggtgcagc tggcggagtc tgggggaggc ttggtcaagc ctggagggtc cctgagactc 60
   tcctgtgcag cctctggatt caccttcagt gactactaca tgagctggat ccgccaggct 120
   ccagggaagg ggctggagtg ggtttcatac attagtacta gtggtagtag catatactat 180
   gtagactctg tgaagggccg attcaccatc tccagggaca acgccaagaa ctcactgtat 240
   ctgcaaatgg acagcctgag agacgacgac acggctgttt attactgtgc gagagatctt 300
   cacggtgact atgcctttga ctcctggggc cagggaaccc tggtcaccgt ctcctcaggt 360
   ggaggcggtt caggcggagg tggctctggc ggtggcggat cgtctgagct gactcaggac 420
   cctgctgtgt ctgtggcctt gggacagaca gtcaggatca catgccaagg agacagtctc 480
   agaagttatt atgcaagctg gtaccagcag aagccaggac aggcccctgt acttgtcatc 540
   tatggtaaaa acaaccggcc ctcagggatc ccagaccgat tctctggctc caagtctggc 600
   aactcagcct ccctggacat cagtgggctc cagtctgagg atgaggctga ttattattgt 660
   gcaacatggg atgacaacct gagtggtccg atattcggcg gagggaccaa ggtcaccgtc 720
   ctaggt 726
<210> 106
   <211> 729
   <212> DNA
   <213> Artificial sequence
<220>
   <223> Nucleotidic
<400> 106
   caggtgcagc tgcaggagtc ggggggaggc ttggtacagc ctggggggtc cctgagactc 60
   tcctgtgcag cctctggatt cacctttagc agctatgcca tgagctgggt ccgccaggct 120
   ccagggaagg ggctggagtg ggtctcagct attagtggta gtggtggtag cacatactac 180
   gcagactccg tgaagggccg gttcaccatc tccagagaca attccaagaa cacgctgtat 240
   ctgcaaatga acagcctgag agccgaggac acggccgtat attactgtgc gaaagtgagc 300
   agcagctggt cccattttga ctactggggc cagggaaccc tggtcaccgt ctcctcaggt 360
   ggaggcggtt caggcggagg tggctccggc ggtggcggat cggatgttgt gatgactcag 420
   tctccttcca ccctgtctgc atctgtagga gacagagtca ccatcacttg ccgggccagt 480
   cagtatatta gtaactggtt ggcctggtat cagcagaaac cagggaaagc ccctaagctc 540
   ctgatctata aggcgtctag tttagaaagt ggggtcccat caaggttcag cggcagtgga 600
   tctgggacag agttcactct caccatcagc agtctgcaac ctgaagattt tgcaacttac 660
   tactgtcaag agagttacaa taccccctta ttcactttcg gccctgggac caagctggag 720
   atcaaacgt 729
<210> 107
   <211> 723
   <212> DNA
   <213> Artificial sequence
<220>
   <223> Nucleotidic
<400> 107
   caggtgcagc tggtggagtc tgggggaggc ttagtggagc ctggggggtc cctgagactc      60
   tcctgtgcgg cctctggatt cacctttagc aactatgcca taaactgggt ccgccaggct     120
   ccagggaagg ggctggagtg ggtggccaac atacaccacg atggaaatgg taaatactat     180
   gtggactctg tggagggccg attcaccatc tccagagaca acgccaagaa ttctctgtat     240
   ctgcaaatgg acagcctgag agccgaggac acggccattt attactgtgc gcgagacggc     300
   tacgggggtt accttgactt gtggggccag ggaaccctgg tcaccgtctc ctcaggtgga     360
   ggcggttcag gcggaggtgg ctctggcggt ggcggatcgt ctgagctgac tcaggaccct     420
   gctgtgtctg tggccttggg acagacagtc aggatcacat gccaaggaga cagcctcaga     480
   agctattatg caagctggta ccagcagaag ccaggacagg cccctgtact tgtcatctat     540
   ggtaaaaaca accggccctc agggatccca gaccgattct ctggctccgg ctcaggaaac     600
   acagcttcct tgaccatcac tggggctcag gcggaagatg aggctgacta ttactgtgca     660
   gcatgggatg acagcctgag tggtccggtg ttcggcggag ggaccaaggt caccgtccta     720
   ggt                                                                   723
<210> 108
   <211> 729
   <212> DNA
   <213> Artificial sequence
<220>
   <223> Nucleotidic
<400> 108
   caggtgcagc tgcaggagtc ggggggaggc gtggtccagc ctgggaggtc cctgagactc    60
   tcctgtgcag cctctcgatt caccttcagt agctatgcta tgcactgggt ccgccaggct   120
   ccaggcaagg ggctggagtg ggtggcagtt atatcatatg atggaagcaa taaatactac   180
   gcagactccg tgaagggccg attcaccatc tccagagaca attccaagaa cacgctgtat   240
   ctgcaaatga acagcctgag agctgaggac acggctgtgt attactgtgc gagagatctc   300
   tcggggtacg gtgactaccc tgactactgg ggccagggaa ccctggtcac cgtctcctca   360
   ggtggaggcg gttcaggcgg aggtggctct ggcggtggcg gatcgtctga gctgactcag   420
   gaccctgctg tgtctgtggc cttgggacag acagtcagaa tcacatgcca aggagacagc   480
   ctcagaagct attatgcaag ctggtaccag cagaagccag gacaggcccc tgtacttgtc   540
   atgtatggta gaaacgagcg gccctcaggg gttcctgacc gattctctgg ctccaagtct   600
   ggcacctctg cctccctggc catcagtggc ctccagccag aggatgaggc taattattac   660
   tgtgcagggt gggatgacag cctgactggt ccggtgttcg gcggagggac caagctgacc   720
   gtcctaggt                                                           729
<210> 109
   <211> 15
   <212> PRT
   <213> Artificial sequence
<220>
   <223> Acid amino
<400> 109
   Ser Gly Gly Gly Gly Ser Gly Gly Gly Gly Ser Gly Gly Gly Gly
   1               5                   10                  15
<210> 110
   <211> 45
   <212> DNA
   <213> Artificial sequence
<220>
   <223> Nucleotidic
<400> 110
   tcaggtggag gcggttcagg cggaggtggc tctggcggtg gcgga 45

## Description

La présente invention a pour objet une nouvelle famille d'anticorps dirigés contre le récepteur de la transferrine (anticorps anti-récepteurs de la transferrine) entièrement humains et cytotoxiques, induisant la mort des cellules cancéreuses hématopoïétiques humaines.

La transferrine (Tf) est une protéine sérique de 80 kDa dont le rôle est de fixer le fer soluble. Elle est endocytée dans les cellules suite à sa liaison avec le récepteur de la transferrine (TfR). L'acidification de l'endosome induit un changement conformationnel qui relargue le fer dans le cytosol. Le complexe Tf/TfR est ensuite reexporté à la membrane où le retour à un pH physiologique provoque une dissociation du complexe Tf/TfR.

Le récepteur de la transferrine humaine (TfR), un marqueur de prolifération cellulaire, joue un rôle prépondérant dans l'absorption cellulaire du fer et dans la régulation de la croissance cellulaire. Il a longtemps été considéré comme une cible intéressante pour diverses pathologies, dont le cancer (car il est surexprimé sur de nombreuses cellules fortement proliférantes) et les maladies cérébrales (car il est exprimé sur la barrière hémato-encéphalique), pour des approches thérapeutiques ou diagnostiques.

Ainsi, au cours des 25 dernières années, un certain nombre d'anticorps monoclonaux (AcM) murins anti-récepteurs de la transferrine (anti-TfR) ont été élaborés au moyen de la technologie des hybridomes, et ils ont été testés pour leur effet inhibiteur *in vitro* et parfois *in vivo* sur des modèles murins de cancer (Daniels et al., The transferrin receptor part I or part II, Clin Immunol., 2006*)* ou en tant que véhicules pour un ciblage du cerveau (Pardridge, Drug targeting to the brain, Pharm res 24 : 1733, 2007*).*

En théorie, l'utilisation d'anticorps monoclonaux anti-récepteurs de la transferrine (AcM anti-TfR) cytotoxiques pour l'immunothérapie du cancer aurait dû être limitée par les effets indésirables induits sur les cellules dépendantes du fer telles que les lignées hématopoïétiques fortement proliférantes.

Cependant, un essai de phase I réalisé avec l'AcM 42/6 qui induit une déplétion en fer s'est révélé encourageant car aucun effet secondaire sur les patients n'a été observé lors de l'administration de l'anticorps (Brooks et al., Clin Cancer Res 1 : 1259, 1995).

Toutefois aucune réponse au cancer n'a été observée chez les patients avec l'anticorps 42/6. Il est probable que l'échec de cet essai clinique ait été dû au statut étranger (murin) de l'anticorps humain anti-souris (HAMA) qui a initié la réponse immunitaire chez les patients, et à la rapide dégradation de l'anticorps qui s'en est suivie après les injections répétées.

L'anticorps murin A24, qui est un AcM anti-TfR inhibiteur de croissance, est actif sur des échantillons de formes aiguës et chroniques de leucémie / lymphome à cellules T chez l'adulte *in vitro* (Moura et al., Blood 103 : 1838, 2004 ; Callens et al., Leukemia 22 :42, 2008) ainsi que sur l'établissement de lymphomes du manteau chez la souris (Lepelletier et al., Cancer Res 67 : 1145, 2007). L'AcM A24 interfère avec le ligand naturel du TfR de la transferrine diferrique chargée ou holotransferrine (holo-Tf). L'A24 diminue l'endocytose de l'holo-Tf et par conséquent l'entrée de fer dans les cellules. De plus, l'AcM A24 réduit l'expression des TfR en perturbant le recyclage des TfR à la surface des cellules.

Parmi les anticorps monoclonaux AcM anti-TfR décrits comme cytotoxiques dans la littérature, tous n'inhibent pas la liaison de la transferrine au TfR. Leur action antiproliférative ou cytotoxique résulte soit d'un pontage entre plusieurs récepteurs induisant la dégradation du TfR ou bien par l'enclenchement d'ADCC (cytotoxicité cellulaire dépendante des anticorps) et de CDC (cytotoxicité dépendante du complément) (Daniels et al., The transferrin receptor part I or part II, Clin Immunol., 2006*).* Sachant que le TfR est exprimé à la surface de certaines cellules quiescentes (cellules de la lignée érythroïde, cellules de l'endothélium vasculaire cérébral, hépatocytes, cellules tubulaires rénales), le mode d'action de ces anticorps peut présenter une certaine cytotoxicité envers ces types cellulaires.

L'ensemble des anticorps anti-TfR existants à ce jour sont des anticorps issus d'immunisations d'animaux. En tant qu'agents thérapeutiques ils présentent donc une immunogénicité potentielle non négligeable qui limite leur utilisation.

Il n'existe pas à l'heure actuelle d'anticorps monoclonal anti-TfR sur le marché. Il existe toutefois des études précliniques en cours pour le développement d'un anticorps chimérique anti-TfR développé par la société MAT Biopharma appliqué au mélanôme métastatique. Cependant l'activité anti-tumorale de cet anticorps s'exerce par recrutement d'ADCC (cytotoxicité cellulaire dépendante des anticorps) et de CDC (cytotoxicité dépendante du complément).

Ainsi, les traitements utilisés à ce jour dans les hémopathies malignes reposent en partie sur l'utilisation de chimiothérapies et d'inhibiteurs pharmacologiques dirigés contre l'activité tyrosine kinase de certains récepteurs. Les chimiothérapies sont adaptées en fonction des pathologies et du stade et grade des tumeurs. Les effets secondaires sont importants et sont liés au manque de spécificité du traitement. Les inhibiteurs pharmacologiques s'avèrent être plus spécifiques mais présentent tout de même une toxicité causée par leur liaison à plusieurs familles de récepteurs ou molécules de signalisation intracellulaire à activité enzymatique. De plus on constate chez certains patients l'apparition de résistances en cours des traitements reliée à l'acquisition de mutations.

La publication D1, « J. Mol. Biol. (2000), 301, 1149-1161 », traite d'anticorps de format scFv humain et monomérique, provenant d'une banque de phages capables d'être internalisés dans des cellules tumorales mammaires SKBR-3 et pas dans des cellules normales de même spécificité cellulaire. L'anticorps H7 décrit dans D1, à savoir plus particulièrement le fragment scFv H7 (sc Fv : « format simple chaîne fragment variable »), est capable d'inhiber la liaison de la transferrine au récepteur de la transferrine, ainsi que la croissance des cellules tumorales mammaires SKBR-3. Le fragment scFv H7 réagit de manière compétitive avec l'holotransferrine pour sa liaison avec les cellules cancéreuses SKBR-3.

La publication D2, « Molecular Immunology 44 (2007), 3377-3788 », cite les six anticorps spécifiquement exemplifiés dans la présente demande, leur obtention *in vitro* par phage display sous un format scFv et leur sélection pour leur capacité à être internalisés spécifiquement à l'intérieur de cellules tumorales mammaires SKBR-3.

Ces six anticorps sont décrits pour leur liaison spécifique avec le récepteur de la transferrine (avec rappel des résultats de D1 cités plus haut) et il est fait l'hypothèse d'une efficacité des six anticorps dans l'inhibition de la prolifération cellulaire des cellules tumorales mammaires SKBR-3.

Cependant cette hypothèse sera infirmée dans une publication D3, « Cancer Research, Vol. 70, No. 13, juillet 2010 », où il est indiqué que finalement lesdits anticorps n'inhibent pas la prolifération de ces cellules (page 5500 dernier paragraphe de la colonne de gauche).

La détermination d'anticorps ne constitue donc qu'une première étape dans la recherche de solutions au traitement de pathologies telles que le cancer. En effet, il faut ensuite pouvoir déterminer sur quelles lignées cellulaires tumorales, et donc pour quelles applications thérapeutiques les anticorps obtenus vont pouvoir être actifs, ce qui s'avère souvent difficile, étant donné d'une part le grand nombre de lignées cellulaires et d'autre part leur comportement difficile à prévoir.

Il a maintenant été découvert de manière surprenante par les Inventeurs que les anticorps objets de l'invention présentent un haut potentiel pour le traitement de cancers de type lymphôme ou leucémie ainsi que comme immunosuppresseurs pour les maladies auto-immunes ou la prévention du rejet de greffe.

Un des buts de l'invention est donc de mettre à disposition de nouveaux anticorps thérapeutiques qui ciblent le récepteur de la transferrine humaine (TfR) et qui présentent un haut potentiel pour le traitement du cancer, en particulier de type lymphôme ou leucémie.

Un autre but de l'invention est de mettre à disposition de nouveaux anticorps thérapeutiques qui ciblent le récepteur de la transferrine humaine et qui puissent être utilisés pour le traitement de maladies auto-immunes ou la prévention du rejet de greffe.

Un autre but de l'invention est de proposer de nouveaux anticorps ciblant le récepteur de la transferrine humaine qui puissent être utilisés pour la vectorisation de molécules biologiquement actives, et plus particulièrement d'agents cytotoxiques, au sein de cellules tumorales surexprimant le TfR.

Un autre but de l'invention est de proposer de nouveaux anticorps ciblant le récepteur de la transferrine humaine qui puissent être utilisés pour la vectorisation de molécules diagnostiques, et plus particulièrement pour l'imagerie cérébrale.

Les nouveaux anticorps objets de la présente invention s'avèrent être des molécules à haut potentiel thérapeutique pour les raisons exposées ci-après et répondent à l'ensemble des buts susmentionnés.

En effet les Inventeurs de la présente invention ont mis au point de nouveaux anticorps monoclonaux entièrement humains et cytotoxiques, spécifiques du récepteur de la transferrine (TfR), qui inhibent la liaison de la transferrine (Tf) au récepteur de la transferrine (TfR), et qui privent ainsi les cellules en fer. Plus particulièrement, les anticorps de l'invention induisent une déplétion des cellules en fer (baisse de la concentration en fer intracellulaire), ce qui a pour conséquence une augmentation du récepteur en fer (TfR) à la surface de la cellule.

De façon particulièrement avantageuse les anticorps humains objets de la présente invention présentent une activité antiproliférative et cytotoxique envers des cellules cancéreuses d'origine hématopoïétique *in vitro* ainsi qu*'in vivo.*

La présente invention a plus particulièrement pour objet une nouvelle construction moléculaire caractérisée en ce qu'elle comprend au moins une séquence en acides aminés choisie parmi :
SEQ ID NO : 1, SEQ ID NO : 2, SEQ ID NO : 3, SEQ ID NO : 4, SEQ ID NO : 5, SEQ ID NO : 6, SEQ ID NO : 7, SEQ ID NO : 8, SEQ ID NO : 9, SEQ ID NO : 10, SEQ ID NO : 11, SEQ ID NO : 12, SEQ ID NO : 13, SEQ ID NO : 14, SEQ ID NO : 15, SEQ ID NO : 16, SEQ ID NO : 17, SEQ ID NO : 18, SEQ ID NO : 19, SEQ ID NO : 20, SEQ ID NO : 21, SEQ ID NO : 22, SEQ ID NO : 23, SEQ ID NO : 24, SEQ ID NO : 25, SEQ ID NO : 26, SEQ ID NO : 27, SEQ ID NO : 28, SEQ ID NO : 29, SEQ ID NO : 30, SEQ ID NO : 31, SEQ ID NO : 32, SEQ ID NO : 33, SEQ ID NO : 34, SEQ ID NO : 35 et SEQ ID NO : 36,
ladite séquence en acides aminés correspondant à une région déterminant la complémentarité avec l'antigène ("CDR" : « complementarity determining region ») du domaine variable de la chaîne lourde (CDR-H) ou de la chaîne légère (CDR-L) d'un anticorps ciblant le récepteur de la transferrine humaine (TfR).

Par construction moléculaire on entend toute construction (telle que par exemple une molécule, un anticorps ou un fragment d'anticorps ou autre), qui pourrait être préparée par l'homme du métier et qui comprendrait une des quelconques séquences en acides aminés SEQ ID NO : 1 à 36 telles que définies dans la présente demande.

La construction moléculaire de l'invention est plus particulièrement constituée par au moins l'une des séquences en acides aminés choisie parmi : SEQ ID NO : 3, SEQ ID NO : 6, SEQ ID NO : 9, SEQ ID NO : 12, SEQ ID NO : 15, SEQ ID NO : 18, SEQ ID NO : 21, SEQ ID NO : 24, SEQ ID NO : 27, SEQ ID NO : 30, SEQ ID NO : 33 et SEQ ID NO : 36.

Les séquences ainsi définies correspondent plus particulièrement aux CDR qui se trouvent en position numérotée 3 dans chaque chaîne lourde (CDR3-H) ou dans chaque chaîne légère (CDR3-L) d'un anticorps ciblant le récepteur de la transferrine humaine (TfR).

Plus particulièrement, les séquences SEQ ID NO : 3, SEQ ID NO : 9, SEQ ID NO : 15, SEQ ID NO : 21, SEQ ID NO : 27 et SEQ ID NO : 33 correspondent aux « CDR3-H » et les séquences SEQ ID NO : 6, SEQ ID NO : 12, SEQ ID NO : 18, SEQ ID NO : 24, SEQ ID NO : 30 et SEQ ID NO : 36 correspondent aux « CDR3-L ».

Selon l'invention, l'une au moins des séquences SEQ ID NO: 1 à SEQ ID NO: 36 de la construction moléculaire est comprise dans l'une des séquences en acides aminés choisies parmi :
SEQ ID NO : 37, SEQ ID NO : 38, SEQ ID NO : 39, SEQ ID NO : 40, SEQ ID NO : 41, SEQ ID NO : 42, SEQ ID NO : 43, SEQ ID NO : 44, SEQ ID NO : 45, SEQ ID NO : 46, SEQ ID NO : 47 et SEQ ID NO:48,
chacune desdites séquences SEQ ID NO : 37 à SEQ ID NO : 48 correspondant au domaine variable de la chaîne lourde (VH) ou de la chaîne légère (VL) d'un anticorps ciblant le récepteur de la transferrine humaine (TfR),
ladite séquence SEQ ID NO : 37 comprenant les séquences SEQ ID NO : 1, SEQ ID NO : 2 et SEQ ID NO : 3,
ladite séquence SEQ ID NO : 38 comprenant les séquences SEQ ID NO : 4, SEQ ID NO : 5 et SEQ ID NO : 6,
ladite séquence SEQ ID NO : 39 comprenant les séquences SEQ ID NO : 7, SEQ ID NO : 8 et SEQ ID NO : 9,
ladite séquence SEQ ID NO : 40 comprenant les séquences SEQ ID NO : 10, SEQ ID NO : 11 et SEQ ID NO : 12,
ladite séquence SEQ ID NO : 41 comprenant les séquences SEQ ID NO : 13, SEQ ID NO : 14 et SEQ ID NO : 15,
ladite séquence SEQ ID NO : 42 comprenant les séquences SEQ ID NO : 16, SEQ ID NO : 17 et SEQ ID NO : 18,
ladite séquence SEQ ID NO : 43 comprenant les séquences SEQ ID NO : 19, SEQ ID NO : 20 et SEQ ID NO : 21,
ladite séquence SEQ ID NO : 44 comprenant les séquences SEQ ID NO : 22, SEQ ID NO : 23 et SEQ ID NO : 24,
ladite séquence SEQ ID NO : 45 comprenant les séquences SEQ ID NO : 25, SEQ ID NO : 26 et SEQ ID NO : 27,
ladite séquence SEQ ID NO : 46 comprenant les séquences SEQ ID NO : 28, SEQ ID NO : 29 et SEQ ID NO : 30,
ladite séquence SEQ ID NO : 47 comprenant les séquences SEQ ID NO : 31, SEQ ID NO : 32 et SEQ ID NO : 33,
ladite séquence SEQ ID NO : 48 comprenant les séquences SEQ ID NO : 34, SEQ ID NO : 35 et SEQ ID NO : 36.

De même selon l'invention, l'une au moins des séquences SEQ ID NO : 37 à SEQ ID NO : 48 est comprise dans l'une des séquences en acides aminés choisies parmi :
SEQ ID NO : 49, SEQ ID NO : 50, SEQ ID NO : 51, SEQ ID NO : 52, SEQ ID NO : 53 et SEQ ID NO : 54,
chacune desdites séquences SEQ ID NO : 49 à SEQ ID NO : 54 comprenant le domaine variable de la chaîne lourde (VH) et de la chaîne légère (VL) d'un anticorps ciblant le récepteur de la transferrine humaine (TfR),
ladite séquence SEQ ID NO : 49 comprenant les séquences SEQ ID NO : 37 et SEQ ID NO : 38 reliées entre elles par le peptide de liaison présentant une séquence en acides aminés SEQ ID NO : 109,
ladite séquence SEQ ID NO : 50 comprenant les séquences SEQ ID NO : 39 et SEQ ID NO : 40 reliées entre elles par le peptide de liaison SED ID NO : 109,
ladite séquence SEQ ID NO : 51 comprenant les séquences SEQ ID NO : 41 et SEQ ID NO : 42 reliées entre elles par le peptide de liaison SED ID NO : 109,
ladite séquence SEQ ID NO : 52 comprenant les séquences SEQ ID NO : 43 et SEQ ID NO : 44 reliées entre elles par le peptide de liaison SED ID NO : 109,
ladite séquence SEQ ID NO : 53 comprenant les séquences SEQ ID NO : 45 et SEQ ID NO : 46 reliées entre elles par le peptide de liaison SED ID NO : 109,
ladite séquence SEQ ID NO : 54 comprenant les séquences SEQ ID NO : 47 et SEQ ID NO : 48 reliées entre elles par le peptide de liaison SEQ ID NO : 109.

Les différentes séquences SEQ ID NO : 37 à SEQ ID NO : 48 susmentionnées pourraient aussi être liées entres elles par un peptide qui présenterait une séquence différente de SEQ ID NO : 109.

Si la taille du peptide de liaison est par exemple diminuée, on obtiendrait alors des molécules de type « diabodies ». Cependant on peut également envisager d'augmenter la taille du peptide de liaison.

Les séquences SEQ ID NO : 49 à SEQ ID NO : 54 comprennent chacune la totalité du domaine variable de la chaîne lourde et de la chaîne légère d'un anticorps ciblant le récepteur de la transferrine humaine (TfR).

Les séquences SEQ ID NO : 37, SEQ ID NO : 39, SEQ ID NO : 41, SEQ ID NO : 43, SEQ ID NO : 45 et SEQ ID NO : 47 définissent chacune la totalité du domaine variable de la chaîne lourde (VH) d'un anticorps ciblant le récepteur de la transferrine humaine (TfR).

Les séquences SEQ ID NO : 38, SEQ ID NO : 40, SEQ ID NO : 42, SEQ ID NO : 44, SEQ ID NO : 46 et SEQ ID NO : 48 définissent chacune la totalité du domaine variable de la chaîne légère (VL) d'un anticorps ciblant le récepteur de la transferrine humaine (TfR).

Les SEQ ID NO : 1 à 3, 7 à 9, 13 à 15, 19 à 21, 25 à 27 et 31 à 33 définissent chacune une région déterminant la complémentarité avec l'antigène du domaine variable de la chaîne lourde (CDR-H) d'un anticorps ciblant le récepteur de la transferrine humaine (TfR).

Les SEQ ID NO : 4 à 6, 10 à 12, 16 à 18, 22 à 24, 28 à 30 et 34 à 36 définissent chacune une région déterminant la complémentarité avec l'antigène du domaine variable de la chaîne légère (CDR-L) d'un anticorps ciblant le récepteur de la transferrine humaine (TfR).

Ainsi, les séquences SEQ ID NO : 1 à SEQ ID NO : 54 concernent la totalité ou une partie seulement du domaine variable de la chaîne lourde et/ou légère d'un anticorps ciblant le récepteur de la transferrine humaine (TfR).

Selon un mode de réalisation avantageux de l'invention, la construction moléculaire telle que définie ci-dessus se présente sous la forme d'un monomère.

Selon un autre mode de réalisation avantageux de l'invention, la construction moléculaire telle que définie ci-dessus se présente sous la forme d'un dimère.

Selon encore un autre mode de réalisation avantageux de l'invention, la construction moléculaire telle que définie ci-dessus comporte en outre un fragment Fc.

De façon préférée selon l'invention, la construction moléculaire telle que définie ci-dessus est plus particulièrement un anticorps ou un fragment d'anticorps.

Les séquences SEQ ID NO : 49 à SEQ ID NO : 54 telles que définies dans la présente demande définissent plus précisément des fragments d'anticorps et non des anticorps « complets » puisque ces séquences ne comprennent pas le domaine constant retrouvé dans les anticorps natifs (immunoglobines).

De manière générale, un anticorps diffère d'un autre par la séquence primaire de son domaine variable de la chaîne lourde (VH) et de la chaîne légère (VL). La séquence du domaine constant est identique pour une classe ou une sous-classe donnée d'anticorps.

Ainsi, pour être tout à fait précis, le terme « anticorps » tel qu'utilisé ci-dessus et ci-dessous pour définir l'objet de la présente invention doit plus particulièrement être compris comme « fragment d'anticorps ».

Les anticorps ou fragments d'anticorps de l'invention sont tous spécifiques du TfR mais n'ont pas les mêmes séquences primaires (voir SEQ ID NO : 49 à SEQ ID NO : 54). Lesdits anticorps diffèrent par leur paratope, à savoir la zone de contact de l'épitope sur l'antigène, en l'occurrence le TfR.

La présente invention a également pour objet les séquences nucléotidiques caractérisées en ce qu'elles codent respectivement pour les séquences en acides aminés SEQ ID NO : 1 à SEQ ID NO : 36 telles que définies ci-dessus et en ce qu'elles sont représentées respectivement par les séquences SEQ ID NO : 55 à SEQ ID NO : 90.

Ainsi SEQ ID NO : 55 représente la séquence nucléotidique codant pour la séquence en acides aminés SEQ ID NO : 1 et ainsi de suite (SEQ ID NO : 56 codant pour SEQ ID NO : 2, SEQ ID NO : 57 codant pour SEQ ID NO : 3, SEQ ID NO : 58 codant pour SEQ ID NO : 4, SEQ ID NO : 59 codant pour SEQ ID NO : 5, SEQ ID NO : 60 codant pour SEQ ID NO : 6, SEQ ID NO : 61 codant pour SEQ ID NO : 7, SEQ ID NO : 62 codant pour SEQ ID NO : 8, SEQ ID NO : 63 codant pour SEQ ID NO : 9, SEQ ID NO : 64 codant pour SEQ ID NO : 10, SEQ ID NO : 65 codant pour SEQ ID NO : 11, SEQ ID NO : 66 codant pour SEQ ID NO : 12, SEQ ID NO : 67 codant pour SEQ ID NO : 13, SEQ ID NO : 68 codant pour SEQ ID NO : 14, SEQ ID NO : 69 codant pour SEQ ID NO : 15, SEQ ID NO : 70 codant pour SEQ ID NO : 16, SEQ ID NO : 71 codant pour SEQ ID NO : 17, SEQ ID NO : 72 codant pour SEQ ID NO : 18, SEQ ID NO : 73 codant pour SEQ ID NO : 19, SEQ ID NO : 74 codant pour SEQ ID NO : 20, SEQ ID NO : 75 codant pour SEQ ID NO : 21, SEQ ID NO : 76 codant pour SEQ ID NO : 22, SEQ ID NO : 77 codant pour SEQ ID NO : 23, SEQ ID NO : 78 codant pour SEQ ID NO : 24, SEQ ID NO : 79 codant pour SEQ ID NO : 25, SEQ ID NO : 80 codant pour SEQ ID NO : 26, SEQ ID NO : 81codant pour SEQ ID NO : 27, SEQ ID NO : 82 codant pour SEQ ID NO : 28, SEQ ID NO : 83 codant pour SEQ ID NO : 29, SEQ ID NO : 30 codant pour SEQ ID NO : 84, SEQ ID NO : 31 codant pour SEQ ID NO : 85, SEQ ID NO : 32 codant pour SEQ ID NO : 86, SEQ ID NO : 33 codant pour SEQ ID NO : 87, SEQ ID NO : 34 codant pour SEQ ID NO : 88, SEQ ID NO : 35 codant pour SEQ ID NO : 89 et SEQ ID NO : 36 codant pour SEQ ID NO : 90).

La présente invention a également pour objet les séquences nucléotidiques caractérisées en ce qu'elles codent respectivement pour les séquences en acides aminés SEQ ID NO : 37 à SEQ ID NO : 48 telles que définies ci-dessus et en ce qu'elles sont représentées respectivement par les séquences SEQ ID NO : 91 à SEQ ID NO : 102.

Ainsi SEQ ID NO : 91 représente la séquence nucléotidique codant pour la séquence en acides aminés SEQ ID NO : 37 et ainsi de suite (SEQ ID NO : 92 codant pour SEQ ID NO : 38, SEQ ID NO : 93 codant pour SEQ ID NO : 39, SEQ ID NO : 94 codant pour SEQ ID NO : 40, SEQ ID NO : 95 codant pour SEQ ID NO : 41, SEQ ID NO : 96 codant pour SEQ ID NO : 42, SEQ ID NO : 97 codant pour SEQ ID NO : 43, SEQ ID NO : 98 codant pour SEQ ID NO : 44, SEQ ID NO : 99 codant pour SEQ ID NO : 45, SEQ ID NO : 100 codant SEQ ID NO : 46, SEQ ID NO : 101 codant pour SEQ ID NO : 47, SEQ ID NO : 102 codant pour SEQ ID NO : 48).

L'invention concerne aussi les séquences nucléotidiques caractérisées en ce qu'elles codent respectivement pour les séquences en acides aminés SEQ ID NO : 49 à SEQ ID NO : 54 telles que définies ci-dessus et en ce qu'elles sont représentées respectivement par les séquences SEQ ID NO : 103 à SEQ ID NO : 108,
ladite SEQ ID NO : 103 comprenant les séquences SEQ ID NO°: 91 et SEQ ID NO°: 92 reliées entre elles par le peptide de liaison présentant une séquence nucléotidique SEQ ID NO : 110,
ladite SEQ ID NO : 104 comprenant les séquences SEQ ID NO°: 93 et SEQ ID NO°: 94 reliées entre elles par SEQ ID NO : 110,
ladite SEQ ID NO : 105 comprenant les séquences SEQ ID NO°: 95 et SEQ ID NO°: 96 reliées entre elles par SEQ ID NO : 110,
ladite SEQ ID NO : 106 comprenant les séquences SEQ ID NO°: 97 et SEQ ID NO°: 98 reliées entre elles par SEQ ID NO : 110,
ladite SEQ ID NO : 107 comprenant les séquences SEQ ID NO°: 99 et SEQ ID NO°: 100 reliées entre elles par SEQ ID NO : 110,
ladite SEQ ID NO : 108 comprenant les séquences SEQ ID NO°: 10 et SEQ ID NO°: 102 reliées entre elles par SEQ ID NO : 110.
SEQ ID NO : 103 représentant la séquence nucléotidique codant pour la séquence en acides aminés SEQ ID NO : 49 et ainsi de suite (SEQ ID NO : 104 codant pour SEQ ID NO : 50, SEQ ID NO : 105 codant pour SEQ ID NO : 51, SEQ ID NO : 106 codant pour SEQ ID NO : 52, SEQ ID NO : 107 codant pour SEQ ID NO : 53 et SEQ ID NO : 108 codant pour SEQ ID NO : 54).

L'invention concerne encore une molécule d'acide nucléique isolée comprenant au moins une des séquences nucléotidiques SEQ ID NO : 55 à SEQ ID NO : 108 telles que définies ci-dessus.

De même l'invention a encore pour objet :
- un vecteur d'expression comprenant une molécule d'acide nucléique telle que définie ci-dessus,
- une cellule hôte ou un organisme comprenant un vecteur d'expression tel que défini ci-dessus.

Un autre objet de l'invention réside dans une composition pharmaceutique comprenant une quantité thérapeutiquement efficace d'au moins une construction moléculaire telle que définie ci-dessus en association avec un véhicule pharmaceutiquement acceptable.

Ainsi que décrit ci-dessus, la construction moléculaire est de préférence un anticorps ou un fragment d'anticorps.

Selon un mode de réalisation avantageux, la construction moléculaire est utilisée dans ladite composition pharmaceutique pour vectoriser une (ou plusieurs) molécule(s) biologiquement active(s).

Les anticorps de l'invention possèdent des propriétés internalisantes (voir exemple I) qui en font un outil intéressant pour jouer le rôle de vecteurs de délivrance desdites molécules biologiquement actives à l'intérieur de cellules cancéreuses surexprimant le TfR.

Selon un autre mode de réalisation avantageux, la construction moléculaire est utilisée dans ladite composition pharmaceutique pour le ciblage de liposomes (formation « d'immunoliposomes ») ou de nanoparticules chargés d'un (ou plusieurs) agent(s) cytotoxique(s) et/ou d'un (ou plusieurs) agent(s) à visée diagnostique.

L'invention concerne encore une composition pharmaceutique telle que définie ci-dessus pour son utilisation comme médicament dans le traitement de pathologies présentant une sur-expression du TfR.

A titre d'exemple de pathologies présentant une sur-expression du TfR, on pourra citer le cancer, et en particulier du type lymphôme ou leucémie.

Selon un mode de réalisation avantageux de l'invention, le traitement contre le cancer se fait plus particulièrement :
- par action antiproliférative et cytotoxique des anticorps de l'invention envers les cellules cancéreuses *in vitro* et/ou *in vivo,*
   et/ou
- par induction des anticorps de l'invention de la mort des cellules cancéreuses (en les déprivant de fer).

La présente invention a encore pour objet une méthode pour inhiber la prolifération cellulaire de cellules cancéreuses et/ou pour induire la mort desdites cellules, caractérisée en ce qu'elle comprend l'administration à un patient d'au moins substance choisie parmi :
- une construction moléculaire telle que définie ci-dessus,
- une composition pharmaceutique telle que définie ci-dessus,
afin de lier ladite substance auxdites cellules cancéreuses et ainsi de causer l'inhibition de la prolifération et/ou d'induire la mort des cellules cancéreuses.

Un autre objet de la présente invention réside dans une méthode de traitement du cancer caractérisée en ce qu'il comprend l'administration à un patient d'au moins substance choisie parmi :
- une construction moléculaire telle que définie ci-dessus,
- une composition pharmaceutique telle que définie ci-dessus,
afin de lier ladite substance au récepteur de la transferrine des cellules cancéreuses et ainsi de causer l'inhibition de la prolifération et/ou d'induire la mort des cellules cancéreuses.

A titre d'exemple de cellules cancéreuses, on pourra citer les cellules d'origine hématopoïétique.

Un autre exemple de pathologies présentant une sur-expression du TfR est celui des pathologies auto-immunes.

En effet, dans ce cas de figure ce sont les effecteurs cellulaires qui présentent une sur-expression du TfR.

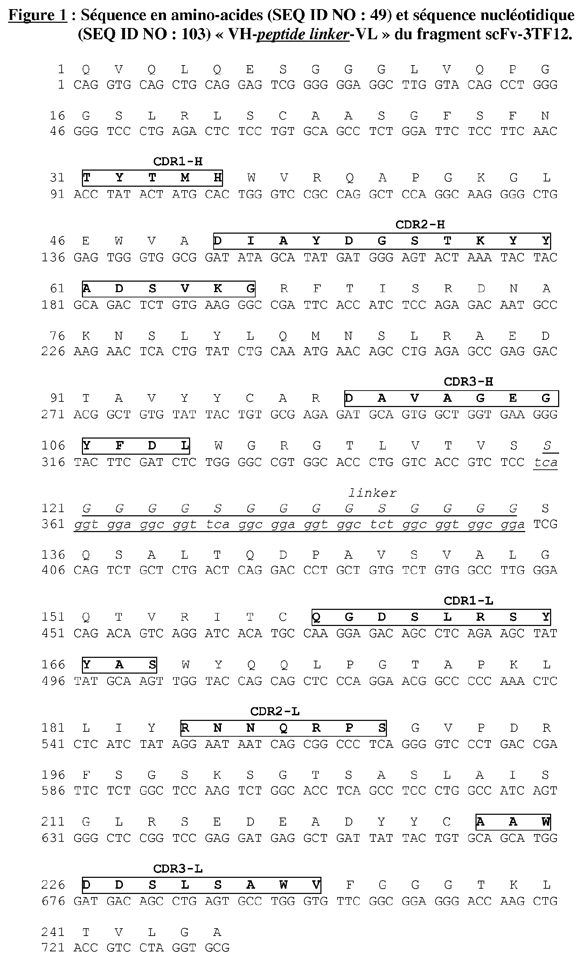

La figure 1 représente la séquence en acides aminés (SEQ ID NO : 49) et la séquence nucléotidique (SEQ ID NO : 103) du domaine variable de la chaîne lourde (VH) et de la chaîne légère (VL) du fragment monovalent de l'invention dénommé « scFv-3TF12 », la chaîne lourde et légère étant reliées entre elles par un peptide de liaison.

Chaque ligne du dessus représente SEQ ID NO: 49 et chaque ligne du dessous représente SEQ ID NO : 103.

Les 3 régions « CDR » de la chaîne lourde (CDR1-H à CDR3-H : SEQ ID NO : 1 à SEQ ID NO : 3) et les 3 régions « CDR » de la chaîne légère (CDR1-L à CDR3-L : SEQ ID NO : 4 à SEQ ID NO : 6) de la séquence SEQ ID NO : 49 sont entourées et représentées en gras.

La chaîne lourde (VH) comprend l'acide aminé n°1 à l'acide aminé n°119 (SEQ ID NO : 37).

La chaîne légère (VL) comprend l'acide aminé n°135 à l'acide aminé n°245 (SEQ ID NO : 38).

Le peptide de liaison (SEQ ID NO : 109) comporte 15 acides aminés n° 120 à 134 qui sont représentés en italique et soulignés (*S G G G G S G G G G S G G G G*).

La séquence nucléotidique SEQ ID NO : 110 qui code pour SEQ ID NO : 109, est en italique, soulignée et est en caractère minuscule.

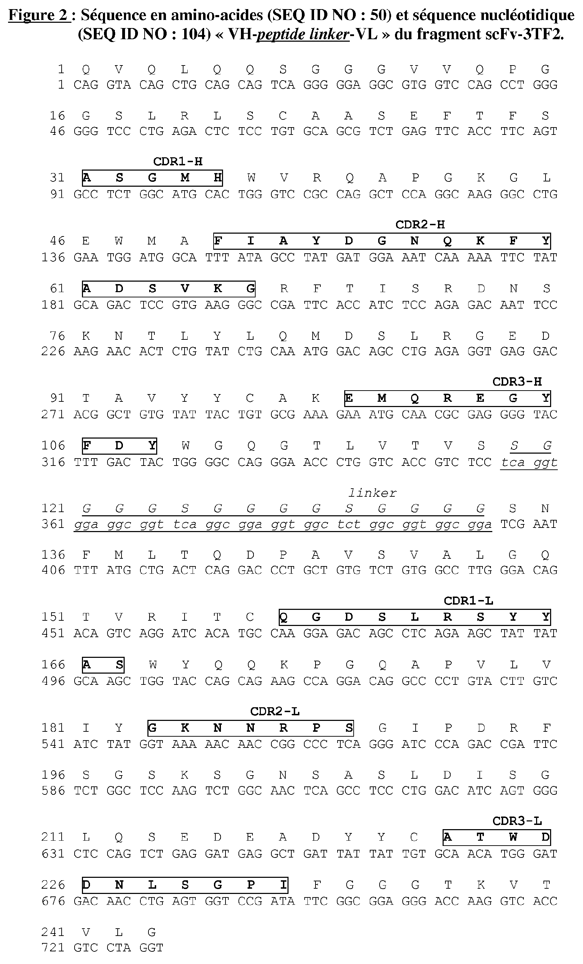

La figure 2 représente la séquence en acides aminés (SEQ ID NO : 50) et la séquence nucléotidique (SEQ ID NO : 104) du domaine variable de la chaîne lourde (VH) et de la chaîne légère (VL) du fragment monovalent de l'invention dénommé « scFv-3TF2 », la chaîne lourde et légère étant reliées entre elles par un peptide de liaison.

Chaque ligne du dessus représente SEQ ID NO: 50 et chaque ligne du dessous représente SEQ ID NO : 104.

Les 3 régions « CDR » de la chaîne lourde (CDR1-H à CDR3-H : SEQ ID NO : 7 à SEQ ID NO : 9) et les 3 régions « CDR » de la chaîne légère (CDR1-L à CDR3-L : SEQ ID NO : 10 à SEQ ID NO : 12) de la séquence SEQ ID NO : 50 sont entourées et représentées en gras.

La chaîne lourde (VH) comprend l'acide aminé n°1 à l'acide aminé n°118 (SEQ ID NO : 39).

La chaîne légère (VL) comprend l'acide aminé n°134 à l'acide aminé n°243 (SEQ ID NO : 40).

Le peptide de liaison (SEQ ID NO : 109) est tel que défini à la figure 1 : il comprend 15 acides aminés allant de l'acide aminé n°119 à 133.

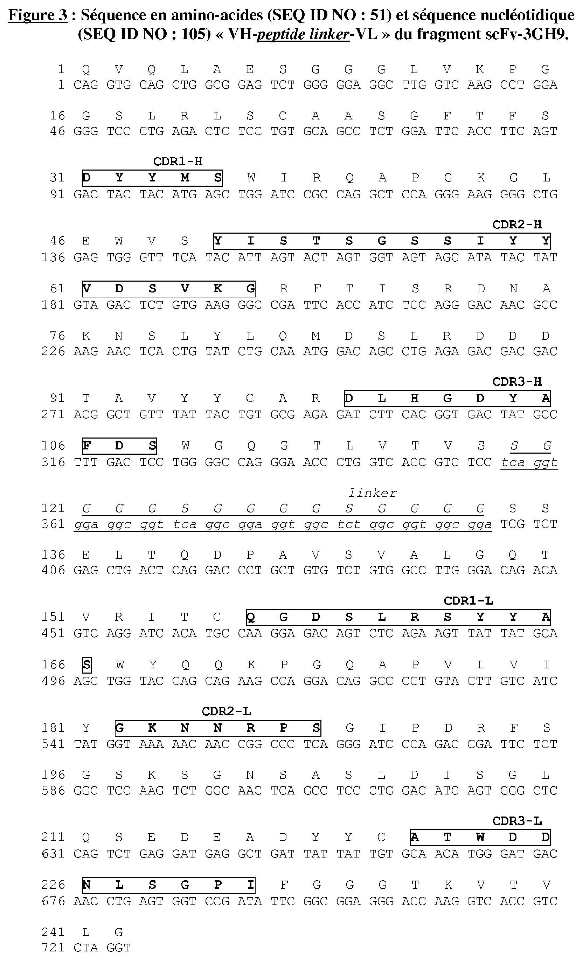

La figure 3 représente la séquence en acides aminés (SEQ ID NO : 51) et la séquence nucléotidique (SEQ ID NO : 105) du domaine variable de la chaîne lourde (VH) et de la chaîne légère (VL) du fragment monovalent de l'invention dénommé « scFv-3GH9 », la chaîne lourde et légère étant reliées entre elles par un peptide de liaison.

Chaque ligne du dessus représente SEQ ID NO: 51 et chaque ligne du dessous représente SEQ ID NO : 105.

Les 3 régions « CDR » de la chaîne lourde (CDR1-H à CDR3-H : SEQ ID NO : 13 à SEQ ID NO : 15) et les 3 régions « CDR » de la chaîne légère (CDR1-L à CDR3-L : SEQ ID NO : 16 à SEQ ID NO : 18) de la séquence SEQ ID NO : 51 sont entourées et représentées en gras.

La chaîne lourde (VH) comprend l'acide aminé n°1 à l'acide aminé n°118 (SEQ ID NO : 41).

La chaîne légère (VL) comprend l'acide aminé n°134 à l'acide aminé n°242 (SEQ ID NO : 42).

Le peptide de liaison (SEQ ID NO : 109) est tel que défini à la figure 1 : il comprend quinze acides aminés allant de l'acide aminé n°119 à 133.

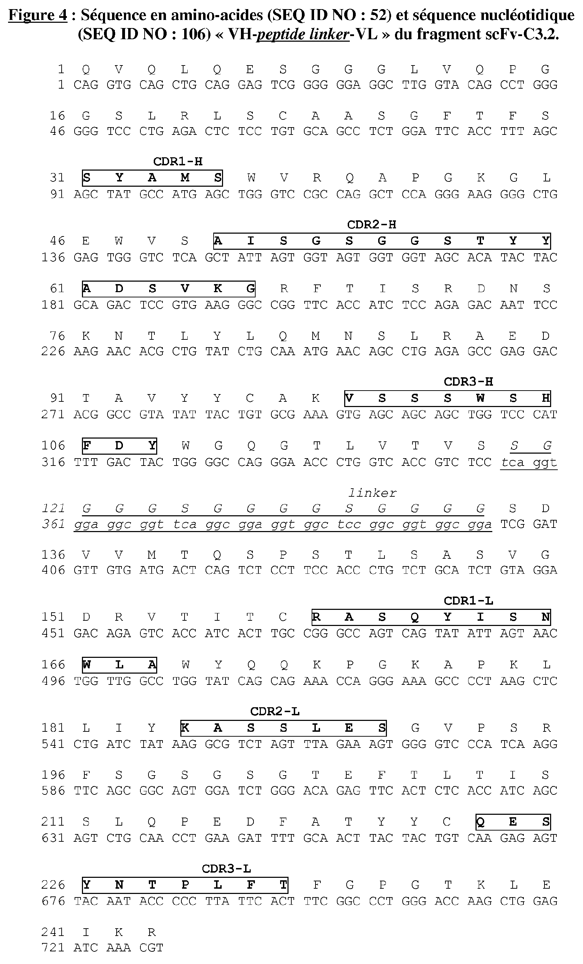

La figure 4 représente la séquence en acides aminés (SEQ ID NO : 52) et la séquence nucléotidique (SEQ ID NO : 106) du domaine variable de la chaîne lourde (VH) et de la chaîne légère (VL) du fragment monovalent de l'invention dénommé « scFv-C3.2 », la chaîne lourde et légère étant reliées entre elles par un peptide de liaison.

Chaque ligne du dessus représente SEQ ID NO: 52 et chaque ligne du dessous représente SEQ ID NO : 106.

Les 3 régions « CDR » de la chaîne lourde (CDR1-H à CDR3-H : SEQ ID NO : 19 à SEQ ID NO : 21) et les 3 régions « CDR » de la chaîne légère (CDR1-L à CDR3-L : SEQ ID NO : 22 à SEQ ID NO : 24) de la séquence SEQ ID NO : 52 sont entourées et représentées en gras.

La chaîne lourde (VH) comprend l'acide aminé n°1 à l'acide aminé n°118 (SEQ ID NO : 43).

La chaîne légère (VL) comprend l'acide aminé n°134 à l'acide aminé n°243 (SEQ ID NO : 44).

Le peptide de liaison (SEQ ID NO : 109) est tel que défini à la figure 1 : il comprend 15 acides aminés allant de l'acide aminé n°119 à 133.

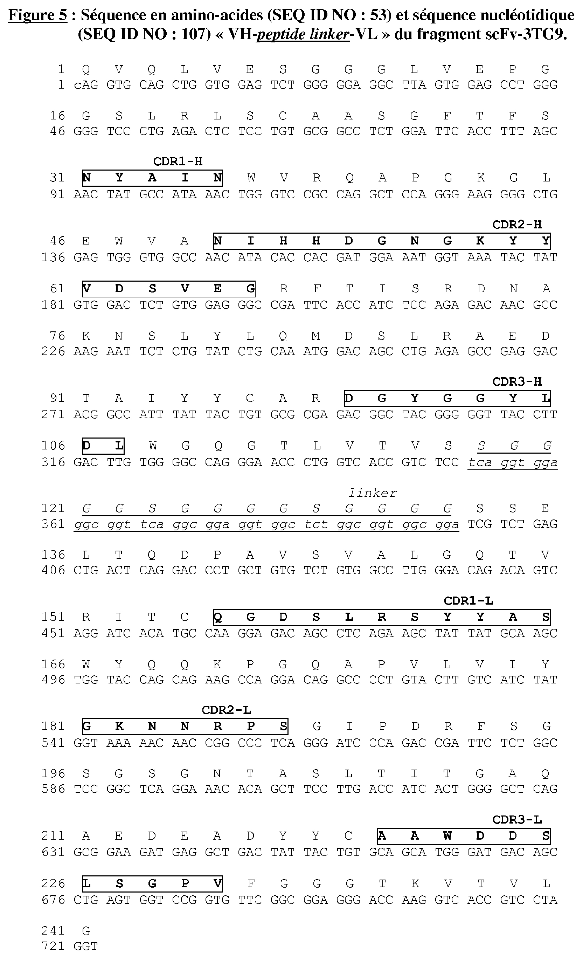

La figure 5 représente la séquence en acides aminés (SEQ ID NO : 53) et la séquence nucléotidique (SEQ ID NO : 107) du domaine variable de la chaîne lourde (VH) et de la chaîne légère (VL) du fragment monovalent de l'invention dénommé « scFv-3TG9 », la chaîne lourde et légère étant reliées entre elles par un peptide de liaison.

Chaque ligne du dessus représente SEQ ID NO: 53 et chaque ligne du dessous représente SEQ ID NO : 107.

Les 3 régions « CDR » de la chaîne lourde (CDR1-H à CDR3-H : SEQ ID NO : 25 à SEQ ID NO : 27) et les 3 régions « CDR » de la chaîne légère (CDR1-L à CDR3-L : SEQ ID NO : 28 à SEQ ID NO : 30) de la séquence SEQ ID NO : 53 sont entourées et représentées en gras.

La chaîne lourde (VH) comprend l'acide aminé n°1 à l'acide aminé n°117 (SEQ ID NO : 45).

La chaîne légère (VL) comprend l'acide aminé n°133 à l'acide aminé n°241 (SEQ ID NO : 46).

Le peptide de liaison (SEQ ID NO : 109) est tel que défini à la figure 1 : il comprend de l'acide aminé n°118 à 132.

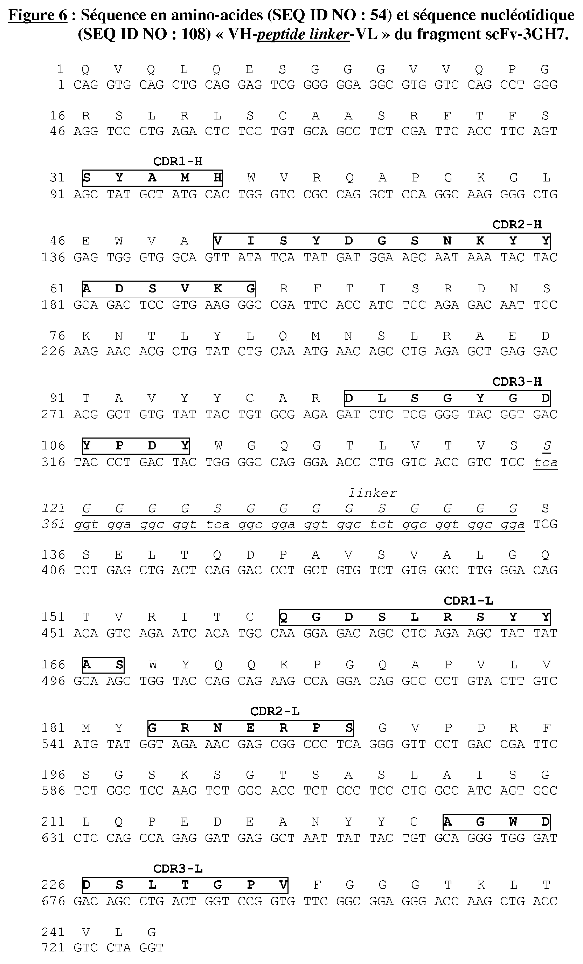

La figure 6 représente la séquence en acides aminés (SEQ ID NO : 54) et la séquence nucléotidique (SEQ ID NO : 108) du domaine variable de la chaîne lourde (VH) et de la chaîne légère (VL) du fragment monovalent de l'invention dénommé « scFv-3GH7 », la chaîne lourde et légère étant reliées entre elles par un peptide de liaison.

Chaque ligne du dessus représente SEQ ID NO: 54 et chaque ligne du dessous représente SEQ ID NO : 108.

Les 3 régions « CDR » de la chaîne lourde (CDR1-H à CDR3-H : SEQ ID NO : 31 à SEQ ID NO : 33) et les 3 régions « CDR » de la chaîne légère (CDR1-L à CDR3-L : SEQ ID NO : 34 à SEQ ID NO : 36) de la séquence SEQ ID NO : 54 sont entourées et représentées en gras.

La chaîne lourde (VH) comprend l'acide aminé n°1 à l'acide aminé n°119 (SEQ ID NO : 47).

La chaîne légère (VL) comprend l'acide aminé n°135 à l'acide aminé n°243 (SEQ ID NO : 48).

Le peptide de liaison (SEQ ID NO : 109) est tel que défini à la figure 1 : il comprend 15 acides aminés allant de l'acide aminé n°120 à 134.

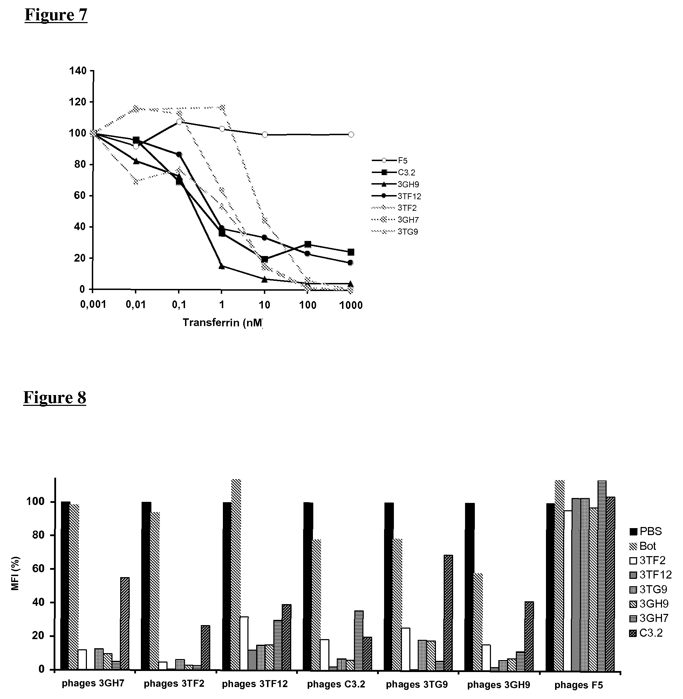

La figure 7 illustre la spécificité des six anticorps de l'invention pour le TfR de séquence SEQ ID N° 49 à SEQ ID N°54, tels que définis précédemment et décrits dans les figures 1 à 6 précédentes.

Des cellules LS-174T ont été incubées une heure à 4°C avec différentes concentrations d'holo-transferrine (Sigma) aux concentrations indiquées.

La liaison des phages-scFv anti-TfR ou du phage scFv-F5 contrôle spécifique du récepteur ErBb2, présent sur les cellules LS-174T, est quantifiée à l'aide d'un anticorps de souris dirigé contre la protéine p8 de la capside phagique (anti-M13, GE healthcare) puis mesurée par FACS à l'aide d'un anticorps fluorescent reconnaissant les immunoglobulines de souris.

Le signal MFI (%) représente l'intensité moyenne de fluorescence (MFI) rapportée à la MFI obtenue en absence de transferrine compétitrice.

La figure 8 représente la cartographie épitopique des épitopes des six anticorps anti-TfR de l'invention (scFvs anti-TfR) de séquence SEQ ID N° 49 à SEQ ID N°54.

Des cellules tumorales LS-174T surexprimant le TfR ont été incubées en présence de 10 µg/ml d'anticorps solubles anti-TfR ou d'un anticorps contrôle dirigé contre la toxine botulique (Bot) pendant une heure à 4°C. 10¹¹ cfu/ml de phages anticorps (phages-scFv) ont ensuite été ajoutés aux cellules pendant une heure à 4°C.

La liaison des phages-scFv anti-TfR ou du phage scFv-F5 contrôle spécifique du récepteur ErBb2 est quantifiée de la même manière que décrit dans la figure 7.

Le signal MFI (%) représente l'intensité moyenne de fluorescence (MFI) rapportée à la MFI obtenue en absence d'anticorps compétiteur.

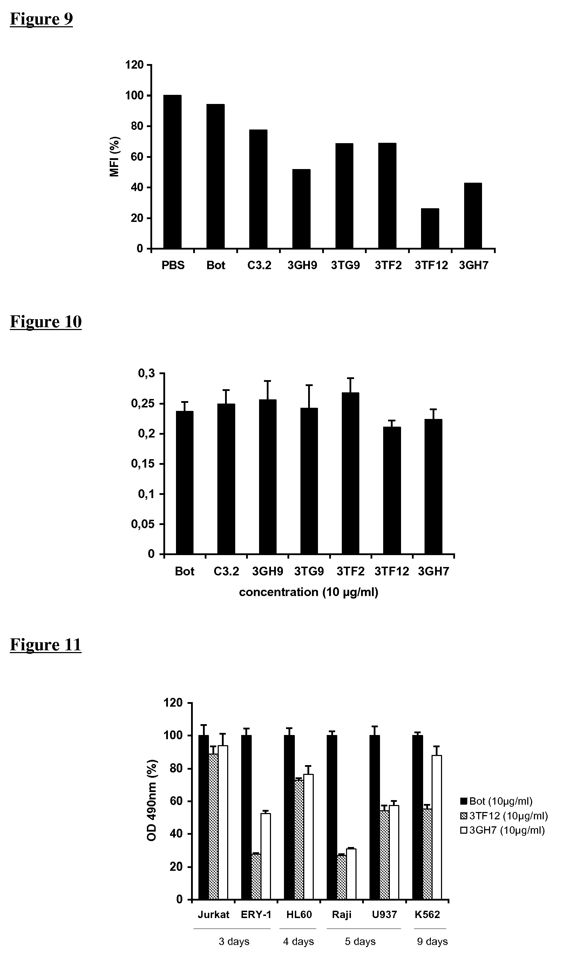

La figure 9 illustre l'inhibition de la liaison Tf/TfR par les six anticorps « scFvs anti-TfR » de l'invention.

Les cellules LS-174T ont été incubées en présence d'anticorps scFv anti-TfR à la concentration de 20 µg/ml pendant une heure à 4°C. Alternativement, un anticorps scFv non spécifique du TfR est utilisé (Bot) ou encore une incubation dans du PBS seulement. Cinq cent nM d'holo-transferrine fluorescente (Tf-FITC) ont ensuite été rajoutés pendant une heure à 4°C. Après un lavage, la Tf-FITC liée aux cellules a été mesurée par FACS. Le signal a été représenté en pourcentage de MFI rapporté à la MFI des cellules incubées sans anticorps (PBS).

La figure 10 illustre le test de prolifération sur la lignée Jurkat (issue d'un lymphome T humain) pendant 3 jours en présence des six anticorps « scFvs anti-TfR » de l'invention.

La figure 11 illustre les tests de viabilité sur plusieurs lignées cancéreuses hématopoïétiques en présence de scFvs anti-TfR, à savoir 3TF12 et 3GH7.

Figures 10 et 11 : une plaque de 96 puits a été ensemencée avec 5 000 cellules Jurkat par puits en sixplicate en milieu RPMI/SVF10% contenant 10 µg/ml d'anticorps solubles. Les cellules ont été incubées en chambre humide CO₂ 5% à 37°C pendant 3 jours. Le nombre de cellules viables a été quantifié à l'aide d'un test MTT (CellTiter 96 Aqueous One Solution Cell Proliferation Assay, Promega).

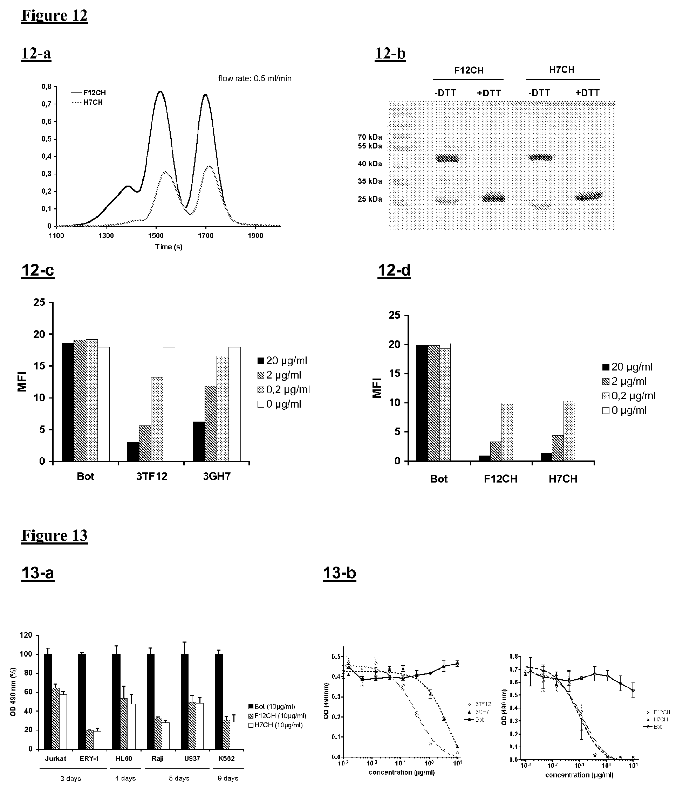

La figure 12 (voir figures 12-a, 12-b, 12-c et 12-d) illustre les résultats obtenus lors de la conversion du format monovalent des anticorps 3TF12 et 3GH7 en format bivalent F12CH et H7CH.

Figures 12-a et 12-b : production d'anticorps scFvCH bivalents (à savoir F12CH et H7CH) à partir du vecteur d'expression bactérien pSYN-HIS-CYS offert par Bin Liu (UCSF). Plus particulièrement la figure 12-a représente le chromatogramme FPLC Superdex 75 représentant un profil d'élution par gel filtration d'anticorps purifiés par chromatographie d'affinité Ni-NTA. La figure 12-b représente une analyse sur gel SDS-PAGE des fractions d'anticorps dimériques isolées après gel filtration (fractions d'environ 55 kDa au niveau du pic à 1500 secondes), en conditions réductrices (+DTT) ou non réductrices (-DTT).

Figures 12-c et 12-d : comparaison des formats dimériques et monomériques des anticorps pour inhiber la liaison de l'holo-Tf aux cellules Raji. Des cellules Raji sont incubées avec des anticorps monovalents (figure 12-c) ou bivalents (figure 12-d) pendant une heure à 4°C aux concentrations indiquées. 500 nM d'holo-Tf-FITC sont ensuite rajoutés aux cellules pendant une heure à 4°C. Après un lavage, la fluorescence est mesurée par FACS.

La figure 13 illustre l'effet anti-prolifératif du format bivalent.

Figure 13-a : plusieurs lignées cellulaires d'origine hématopoïétique sont incubées pendant plusieurs jours en présence d'anticorps solubles bivalents à 10 µg/ml. La viabilité cellulaire est quantifiée à l'aide d'un test MTT (CellTiter 96 Aqueous One Solution Cell Proliferation Assay, Promega ) (OD 490nm).

Figure 13-b : des cellules ERY-1 sont incubées pendant trois jours avec des anticorps anti-TfR solubles monovalents (à gauche) ou bivalents (à droite) aux concentrations indiquées. La viabilité est quantifiée par un test MTT.

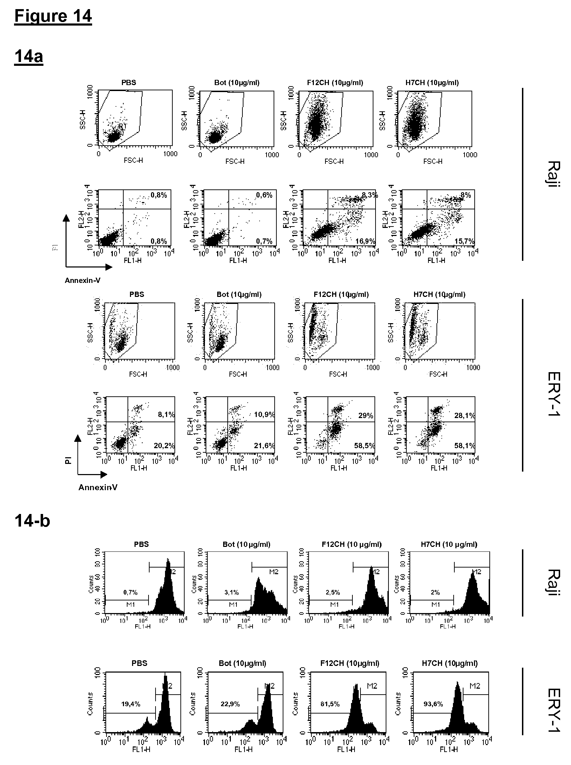

La figure 14 illustre l'induction de la mort cellulaire des lignées Raji et ERY-1 par les anti-TfR F12CH et H7CH.

Plus particulièrement, la figure 14-a illustre le marquage de la translocation des phosphatidyl-sérines. Les lignées Raji (graphique du haut) et ERY-1 (graphique du bas) sont traitées respectivement pendant 4 et 2 jours avec les anticorps F12CH, H7CH et le contrôle négatif Bot à 10 µg/ml. Les cellules ont été lavées puis marquées à l'iodure de propidium (PI) et à l'annexine-V-FITC. Le marquage annexine-V positif, PI négatif correspond aux cellules apoptotiques alors qu'un marquage annexine-V positif, PI positif correspond aux cellules nécrotiques.

La figure 14-b illustre le marquage de la dépolarisation de la membrane mitochondriale. Les lignées cellulaires Raji (graphique du haut) et ERY-1 (graphique du bas) sont traitées de la même façon qu'en 14-a. Après un lavage, les cellules sont marquées avec du 3,3'-dihexyloxacarbocyanine (DIOC6). Une chute d'intensité de fluorescence corrèle avec une chute du potentiel mitochondrial.

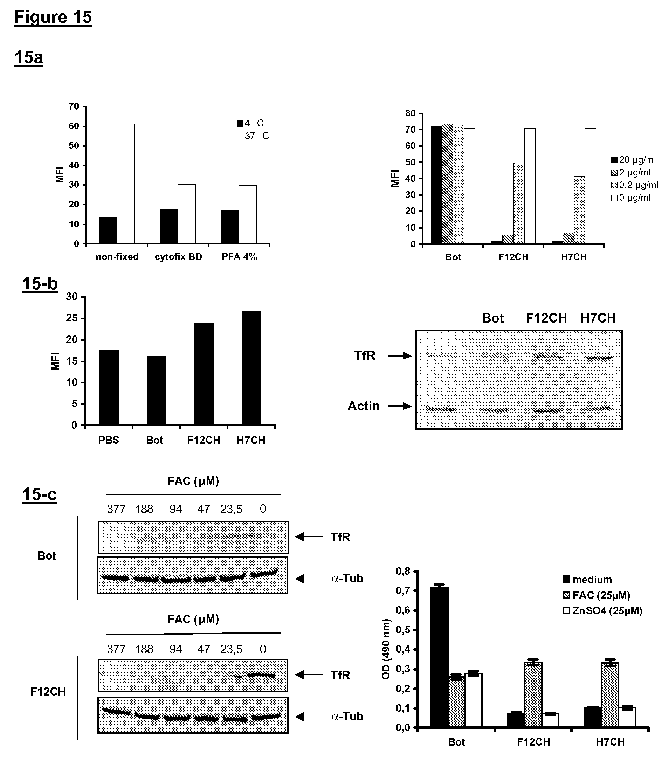

La figure 15 illustre le mécanisme d'action des anti-TfR F12CH et H7CH.

Dans la figure 15-a de gauche, des cellules Raji, fixées ou non fixées, sont incubées en présence de 500 nM de Tf-FITC pendant trois heures à 37°C ou 4°C. Après un lavage, la fluorescence est mesurée par FACS.

Figure 15-a de droite: des cellules Raji sont incubées avec des anticorps aux concentrations indiquées pendant une heure à 37°C avant d'être incubées en présence de 500 nM de Tf-FITC pendant 3 heures. Après un lavage la fluorescence est mesurée par FACS.

Figure 15-b de gauche : des cellules Raji sont incubées pendant 4 jours avec des anticorps à la concentration de 10 µg/ml. Les cellules sont ensuite lavées en tampon glycine (glycine 50 mM, NaCl 500 mM, pH 2.8) avant d'être fixées et marquées par de la Tf-FITC (500 nM) pendant 3 heures à 37°C.

Figure 15-b de droite : des cellules Raji sont incubées pendant 4 jours avec des anticorps à 10 µg/ml. Des extraits protéiques sont ensuite réalisés à l'aide d'un tampon de lyse (Tris-Hcl pH 7.5 50 mM, NaCl 250 mM, EDTA pH 8 10 mM, DTT 1 mM, NP40 1%, inhibiteurs de protéases (Roche)). Après migration sur gel SDS-PAGE les protéines sont transférées sur membrane de nitrocellulose. L'expression du TfR et de l'actine (cette dernière servant de contrôle de charge du gel) est quantifiée à l'aide d'un anticorps de souris anti-TfR (Zymed) et d'un anticorps de chèvre anti-alpha actine (Santa-Cruz). La détection est réalisée grâce à deux anticorps couplés à l'enzyme HRP reconnaissant les immunoglobulines de souris pour le TfR et les immunoglobulines de chèvre pour l'actine.

Figure 15-c de gauche : des cellules Raji sont traitées avec les anticorps F12CH ou Bot à 10 µg/ml pendant 4 jours en présence de citrate d'ammonium ferrique (FAC) aux concentrations indiquées. Les cellules sont ensuite lavées et des extraits protéiques sont réalisés de la même façon que précédemment. Après migration sur gel SDS-PAGE et transfert sur membrane, le TfR est quantifié à l'aide d'un anticorps de souris anti-TfR (Zymed) et l'alpha tubuline (contrôle de charge du gel) à l'aide d'un anticorps de souris anti-alpha tubuline (Santa-Cruz).

Figure 15-c de droite : des cellules ERY-1 sont traitées avec des anticorps à 5 µg/ml en présence de citrate d'ammonium ferrique (FAC) ou de sulfate de zinc (ZnSO₄) à 25 µM. La viabilité cellulaire est quantifié à l'aide d'un test MTT (CellTiter 96 Aqueous One Solution Cell Proliferation Assay, Promega).

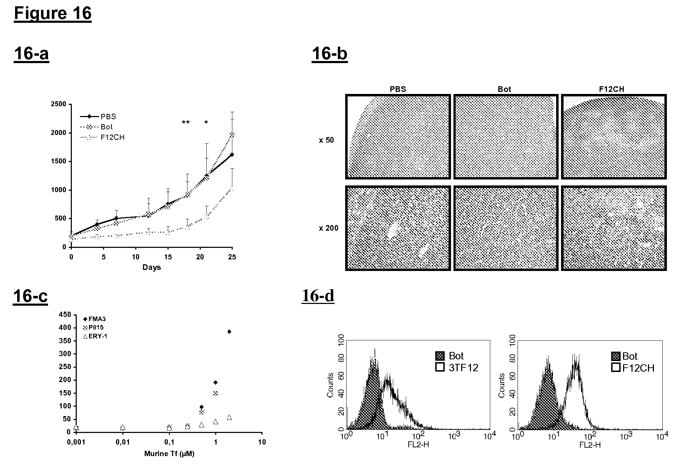

La figure 16 illustre une étude *in vivo* de l'anticorps F12CH.

Figure 16-a : des souris nudes athymiques sont irradiées à 4 Gy avant l'injection sous-cutanée de 2 millions de cellules ERY-1 à chaque animal. Une fois que les tumeurs ont atteint un volume moyen de 200 mm³, les souris sont réparties dans trois cages contenant chacune cinq souris. Un volume de 200 µl de PBS ou contenant 200 µg d'anticorps est ensuite injecté deux fois par semaine par voix intra-péritonéale à chaque souris dans le côté opposé au site de localisation de la tumeur à J₀. La croissance tumorale est mesurée à l'aide d'un pied à coulisse selon la formule : volume = (π/6) x (longueur+largeur)³. Les souris sont sacrifiées à J₂₅. Les différences de moyenne entre les groupes sont analysées à l'aide d'un test de Mann-Whitney (* pour p<0,01, ** pour p<0,005).

Figure 16-b : après prélèvement, les tumeurs sont fixées puis marquées à l'hématoxyline-éosine-safran (HES).

Figure 16-c : des cellules FMA3, P815 (lignées P815 et FMA3 dérivées d'un mastocytôme murin) et ERY-1 sont incubées avec de l'holo-transferrine de souris biotinylée (Tfm) (Rockland) aux concentrations indiquées pendant une heure à 4°C. La liaison de la Tfm aux cellules est détectée à l'aide de streptavidine couplée à de la phycoérythrine (BD Pharmingen). Après un lavage, la fluorescence est mesurée par FACS.

Figure 16-d gauche: des cellules FMA3 sont incubées avec l'anticorps 3TF12 (monomérique) ou l'anticorps Bot à la concentration de 50 µg/ml pendant une heure à 4°C. La liaison des anticorps aux cellules est mesurée à l'aide d'un anticorps secondaire dirigé contre le tag myc (anti c-myc, Sigma) des anticorps 3TF12 et Bot puis d'un anticorps anti-immunoglobuline de souris couplé à la phycoérythrine (BD Pharmingen). Après un lavage, la fluorescence est mesurée par FACS. La courbe grise pleine correspond à la fluorescence du marquage Bot alors que la courbe transparente correspond à celle du marquage 3TF12.

Figure 16-d droite : des cellules FMA3 sont incubées avec l'anticorps F12CH (dimérique) ou l'anticorps Bot à la concentration de 50 µg/ml pendant une heure à 4°C. La liaison des anticorps aux cellules est mesurée à l'aide d'un anticorps secondaire dirigé contre le tag histidine (penta-His antibody, Qiagen) des anticorps F12CH et Bot puis d'un anticorps anti-immunoglobuline de souris couplé à la phycoérythrine (BD Pharmingen). Après un lavage, la fluorescence est mesurée par FACS. La courbe grise pleine correspond à la fluorescence du marquage Bot alors que la courbe transparente correspond à celle du marquage F12CH.

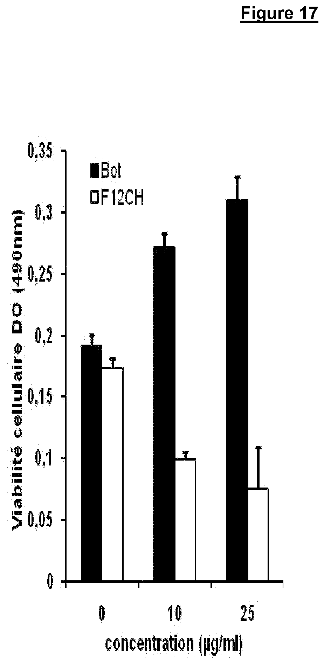

La figure 17 représente un test de prolifération sur des cellules sanguines mononuclées humaines (PBMC) traitées avec F12CH. Les cellules sont ensemencées à 2.10⁴ cellules par puits en milieu RPMI, 10% SVF complété avec de l'IL2 (50 ng/ml). Les cellules sont traitées avec les anticorps Bot ou F12CH (format dimérique produit en bactéries) pendant 3 jours aux concentrations indiquées. La viabilité est mesurée à l'aide d'un test MTT (CellTiter 96 Aqueous One Solution Cell Proliferation Assay, Promega ) (OD 490nm).

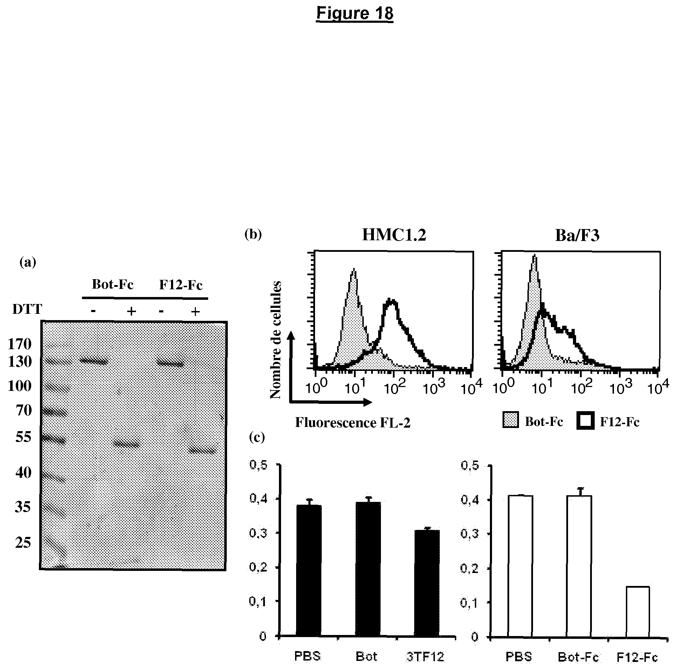

La figure 18 représente l'étude du format scFv-Fc des anticorps Bot-Fc et F12-Fc.

Figure 18 (a) : Analyse sur gel SDS-PAGE de la purification des anticorps Bot-Fc et F12-Fc en condition réductrice et non-réductrice. Un µg d'anticorps a été déposé dans chaque puits du gel.

Figure 18 (b) : Marquage membranaire des cellules humaines HMC1.2 (gauche) et murines Ba/F3 (droite) par les anticorps Bot-Fc (gris) et F12-Fc (noir). Les cellules ont été incubées avec les anticorps à 10 µg/ml pendant 1 heure à 4°C. La liaison des scFv-Fc aux cellules a été détectée à l'aide d'un anticorps secondaire dirigé contre le fragment Fc humain et couplé à la phycoérythrine (Rockland).

Figure 18 (c) : Tests de l'effet des deux formats d'anticorps scFv versus scFv-Fc sur la prolifération de la lignée HMC1.2. Les cellules HMC1.2 (10⁴ par puits, en sixplicate) ont été ensemencées en milieu RPMI, 10% SVF avec les anticorps à 10 µg/ml (soit au format scFv, soit au format scFv-Fc). La viabilité cellulaire a été mesurée au bout de 6 jours (scFv graphique gauche) et 4 jours (graphique droite) par un test MTT (CellTiter 96 Aqueous One Solution Cell Proliferation Assay, Promega), (OD 490nm).

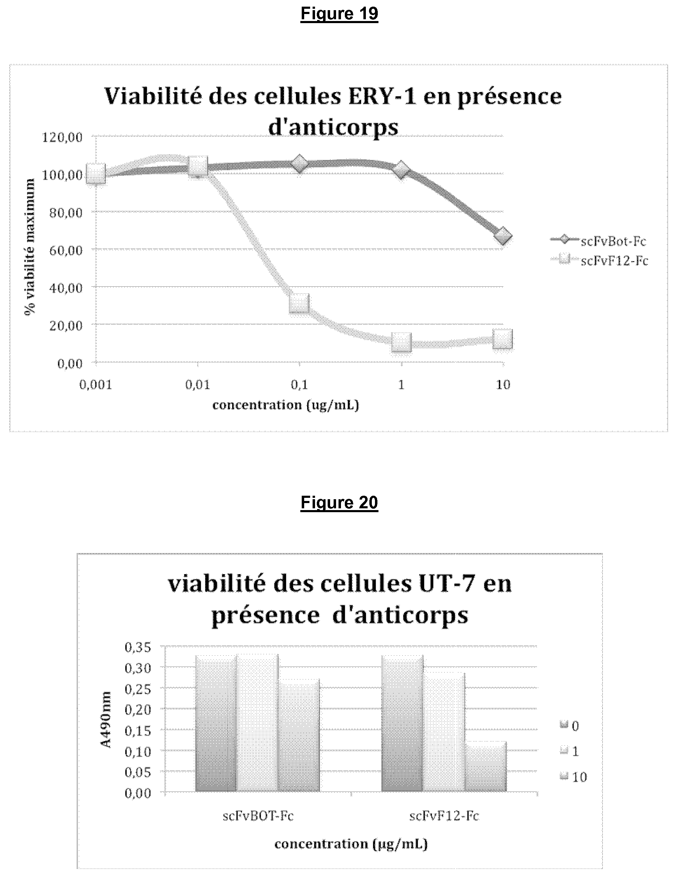

La figure 19 représente l'évaluation de la sensibilité des cellules ERY-1 au format bivalent scFv-Fc pour l'anticorps F12. Une plaque 96 puits a été ensemencée avec 4 000 cellules par puits en sixplicate en milieu RPMI/SVF10% contenant 0,001, 0,01, 0,1, 1, ou 10 µg/ml d'anticorps solubles. Les cellules ont été incubées en chambre humide CO₂ 5% à 37°C pendant 4 jours. Le nombre de cellules viables a été quantifié à l'aide d'un test MTT (CellTiter 96 Aqueous One Solution Cell Proliferation Assay, Promega). Les résultats sont représentés comme le % de viabilité par rapport à un contrôle sans anticorps.

La figure 20 représente l'inhibition de prolifération de la lignée UT-7 en présence d'anticorps 3TF12. Une plaque 96 puits a été ensemencée avec 4 000 cellules par puits en sixplicate en milieu IMDM/SVF10%/Erythropoïétine à 2U/mL contenant 0, 1 ou 10 µg/ml d'anticorps solubles. Les cellules ont été incubées en chambre humide CO₂ 5% à 37°C pendant 4 jours. Le nombre de cellules viables a été quantifié à l'aide d'un test MTT (CellTiter 96 Aqueous One Solution Cell Proliferation Assay, Promega). Les résultats représentent la valeur de l'absorbance à 490 nm en fonction de la concentration en anticorps appliquée.

Les exemples ci-dessous illustrent l'invention, ils ne la limitent en aucune façon.

### Exemple I : Six anticorps anti-TfR différents issus d'une sélection fonctionnelle.

Les anticorps anti-TfR ont été obtenus in vitro par phage display sous un format scFv (format simple chaîne fragment variable) et ont été sélectionnés pour leur capacité à être endocytés (internalisés) spécifiquement à l'intérieur de cellules tumorales mammaires (SKBR3).

Cette sélection a permis d'obtenir 6 anticorps d'un poids moléculaire de 28 kDa chacun, dénommés respectivement 3TF12 (SEQ ID NO : 49), 3TF2 (SEQ ID NO : 50), 3GH9 (SEQ ID NO : 51), C3.2 (SEQ ID NO : 52), 3TG9 (SEQ ID NO : 53) et 3GH7 (SEQ ID NO : 54).

Ces fragments variables simples chaîne (scFv) sont encore appelés fragments monovalents et représentent la forme monomère.

Les anticorps de l'invention sont spécifiques du TfR : la liaison de chaque phage-scFv anti-TfR est inhibée de façon dose- dépendante par la présence d'holo-transferrine (figure 7). Ils reconnaissent des épitopes proches ou communs : la liaison de chaque phage-scFv anti-TfR est inhibée par la présence d'un scFv anti-TfR soluble (figure 8). Ils chevauchent le site de liaison de l'holo-transferrine (holo-Tf) au TfR : chaque scFv anti-TfR inhibe la liaison de l'holo-Tf au TfR de façon plus ou moins prononcée avec un maximum d'inhibition pour 3TF12 et 3GH7 (figure 9).

### Exemple II : Effets anti-prolifératifs des anticorps anti-TfR et étude de leur mécanisme d'action.

Les cellules cancéreuses d'origine hématopoïétique possèdent des besoins en fer élevé. Un test de prolifération a été effectué sur la lignée Jurkat développée à partir d'une leucémie aigüe lymphoblastique T pendant 3 jours en présence des six scFvs anti-TfR.

Seuls les anticorps 3TF12 et 3GH7 présentent une activité anti-proliférative faible mais reproductible (figure 10) sur la lignée Jurkat.

Les anticorps qui présentent un effet anti-prolifératif sur la lignée Jurkat, c'est-à-dire 3TF12 et 3GH7, sont également ceux qui inhibent le mieux la liaison de la Tf au TfR.

Afin de déterminer si cet effet anti-prolifératif est propre aux anticorps ou bien propre à l'origine de la lignée cellulaire utilisée, la viabilité de cinq autres lignées cancéreuses d'origine hématopoïétique a été testée sur plusieurs jours en présence des anticorps 3TF12 ou 3GH7 à 10 µg/ml (figure 11).

Des effets anti-prolifératifs marqués sont observés (jusqu'à 80% d'inhibition sur les lignées Raji et ERY-1) dépendant du type cellulaire testé.

Le changement de format monomérique des anticorps en format dimérique (F12CH pour 3TF12 et H7CH pour 3GH7) a permis d'augmenter l'affinité des anticorps pour le TfR : le format bivalent des anticorps améliore l'inhibition de la Tf au TfR (figures 12-a à 12-d). L'effet anti-prolifératif est amélioré.

Le poids moléculaire des fragments variables dimériques ou bivalents est d'environ 55 kDa.

Ce changement de format a permis de potentialiser l'effet anti-prolifératif des scFvs monovalents (figure 13) sur toutes les lignées testées, ainsi que de diminuer l'IC₅₀ relative à chaque anticorps (tableau 1).

**Tableau 1 : comparaison des IC₅₀ obtenues pour chaque anticorps par un test de prolifération sur la lignée ERY-1 incubée en présence d'anticorps, sous format monomérique ou dimérique, pendant trois jours.**

| Anticorps | IC₅₀ (µg/ml) |
|---|---|
| Monomères | |
| 3TF12 | 0,35 |
| 3GH7 | 4,86 |
| Dimères | |
| F12CH | 0,10 |
| H7CH | 0,09 |

Le format bivalent d'anticorps améliore l'effet antiprolifératif des anti-TfR 3GH7 et 3TF12. Cet effet est observé sur toutes les lignées testées (figure 13).

De plus, les anti-TfR F12CH et H7CH induisent la mort cellulaire par apoptose (pour la lignée ERY-1) ou par autophagie (pour la lignée Raji) (figure 14).

En effet, les anti-TfR induisent le déclenchement d'une mort cellulaire de type apoptotique passant par la voie mitochondriale pour la lignée ERY-1. Après la lignée Raji, on observe après traitement une translocation des phosphatidyl-serines sans chute du potentiel mitochondrial. De plus la granularité des cellules traitées augmente (figure 14-a en haut) ce qui signifierait que ce phénomène de mort cellulaire est relié à un processus autophagique pour celle lignée.

Les Inventeurs de la présente invention ont ensuite découvert le mécanisme d'action responsable de la cytotoxicité de ces anticorps.

Sachant que les anticorps inhibent la liaison de la Tf au TfR à 4°C, les Inventeurs ont voulu déterminer si cette inhibition est maintenue à 37°C. De cette façon il est possible de savoir si l'internalisation de l'holo-Tf est bloquée par les anti-TfR bivalents dans un contexte dynamique.

La Tf-FITC associée aux cellules a d'abord été quantifiée après trois heures d'incubation à 37°C (figure 15-a à gauche). Afin de distinguer le signal relatif à la Tf-FITC internalisée du signal de la Tf-FITC simplement liée en surface, la même expérience a été répétée sur des cellules préalablement incubées dans une solution de fixation (paraformaldéhyde 4% préparé extemporanément ou solution commerciale de fixation, BD). Une réduction du signal de 50% est observée lorsque les cellules sont fixées par rapport aux cellules non fixées. Une partie de la fluorescence associée aux cellules correspond donc à une localisation intracellulaire. Lorsque des cellules sont préalablement incubées avec F12CH ou H7CH à diverses concentrations (0,2 à 20 µg/ml), une inhibition dose-dépendante du signal est observée.

Ce résultat permet de conclure que les anticorps F12CH et H7CH bloquent efficacement l'internalisation de l'holo-Tf dans les cellules.

Les Inventeurs ont ensuite voulu déterminer si la fixation des anticorps F12CH et H7CH sur des cellules cancéreuses entraîne une modification du taux d'expression du TfR.

Pour cela, des cellules Raji sont incubées pendant 4 jours avec les anti-TfR F12CH ou H7CH à 10 µg/ml. Les cellules sont ensuite lavées en tampon glycine (pH 2) pour éliminer les anticorps scFv liés à la surface cellulaire, puis incubées avec de la Tf-FITC pour quantifier l'expression membranaire du TfR (figure 15-b à gauche) à la surface cellulaire.

On constate que les cellules traitées surexpriment le TfR. Lorsque des extraits protéiques sont réalisés à partir des mêmes cellules et que l'expression du TfR est analysée par western-blot (figure 15-b à droite), cette surexpression est également retrouvée dans les extraits traités comparés aux extraits témoins. Il y a donc une augmentation globale de l'expression du TfR.

L'expression du TfR est régulée par la présence de motifs IRE (iron responsive éléments) présents sur l'ARNm du TfR qui le stabilisent lorsque la concentration en fer dans les cellules est faible. Inversement, en cas d'excès de fer, l'ARNm du TfR est dégradé et le TfR n'est plus synthétisé (Daniels *et al.,* The transferrin receptor part I or part II, *Clin Immunol., 2006).*

Pour cette raison, les Inventeurs ont voulu déterminer si la surexpression du TfR observée suite à l'action des anticorps F12CH ou H7CH est liée à une déplétion en fer induite par les anticorps ou bien reliée à un autre mécanisme.

Pour cela, les cellules Raji sont traitées avec l'anticorps contrôle Bot et l'anticorps F12CH pendant 4 jours en présence de fer soluble (FAC, citrate d'ammonium ferrique) dans le milieu de culture à diverses concentrations (figure 15-c à gauche).

On constate que la présence de fer réprime l'expression du TfR dans les deux cas (Bot et F12CH) ce qui prouve que la surexpression du TfR observée pour F12CH est bien liée à une déplétion en fer induite par blocage d'endocytose de la transferrine.

Enfin, les Inventeurs ont voulu déterminer si la cytotoxicité des anticorps est liée à la déplétion en fer qu'ils induisent. La lignée cellulaire la plus sensible (ERY-1) est traitée pendant 3 jours en présence d'anticorps supplémentés par des cations métalliques (fer ou zinc, ce dernier utilisé comme contrôle négatif) et la viabilité cellulaire est quantifiée à l'aide d'un test MTT (figure 15-c à droite). On constate qu'un apport exogène de fer soluble annule la cytotoxicité des anticorps.

En conclusion, la surexpression du TfR par les cellules observée suite à l'action de F12CH est bien liée à une déplétion en fer induite par F12CH.

Cette propriété mécanistique est unique par comparaison au mode d'action d'autres anticorps de souris anti-TfR préalablement décrits. En effet, contrairement aux anticorps de l'invention, ces derniers nécessitent un format multivalent pour exercer leur action cytotoxique, qui corrèle avec une diminution d'expression du TfR au niveau membranaire (IgG A24, (Lepelletier et al., Cancer Res 67 : 1145, 2007 ; Moura et al., Blood 103 : 1838, 2004 ), IgA 42/6 (Taetle et al., Cancer Res 46, 1759-63, 1986), « avidine-fused IgG3 » (Ng et al., Blood 108, 2745-54, 2006), IgM R17 208 (Lesley et Schutle, Mol Cell Biol 5, 1814-21, 1985)).

Les Inventeurs ont ainsi pu déterminer que les anticorps de l'invention induisent une déplétion des cellules en fer ce qui a pour conséquence une augmentation du récepteur en fer (TfR) à la surface de la cellule.

Cependant cette information n'est pas suffisante en soi pour déterminer sur quelles lignées cellulaires les anticorps de l'invention vont être actifs, car il est impossible de déterminer si une lignée cellulaire est sensible à une déprivation en fer. En effet, les raisons de la sensibilité plus ou moins forte à une déprivation en fer ne sont pas connues, et ne dépendent pas en particulier du taux de récepteur en surface (voir figure 2C du document D3 précédemment mentionné).

### Exemple III : Etude d'un modèle murin de tumeur humaine xénogreffée.

Il a été montré dans les exemples précédents que les anticorps F12CH et H7CH possèdent une activité anticancéreuse prononcée sur plusieurs lignées cellulaires d'origine hématopoïétique *in vitro.*

Afin de déterminer si cette activité anticancéreuse de l'anticorps F12CH est maintenue dans un environnement tumoral *in vivo,* un modèle de xénogreffe sous-cutanée a été développé chez la souris nude à partir de la lignée cellulaire ERY-1. Une fois la prise de greffe réalisée (volume tumoral moyen de 200 mm³), 200 µg d'anticorps F12CH ou Bot ont été injectés à chaque animal deux fois par semaine.

La croissance tumorale a été mesurée sur 25 jours et est représentée sur la figure 16-a.

On constate que l'anticorps F12CH ralentit la croissance tumorale en comparaison du véhicule contrôle (injection de tampon PBS seul) et du contrôle isotypique (injection d'un anticorps non spécifique produit dans les mêmes conditions).

Dans les coupes de tumeurs des souris traitées (figure 16-b), on peut noter la présence de fibrose et de cellules pré-nécrotiques.

L'effet observé est d'autant plus pertinent physiologiquement qu'il a été démontré que l'holo Tf murine, présente dans l'hôte à des concentrations de l'ordre de 10 mM, présente une réactivité croisée pour le TfR humain (figure 16-c) et que l'anticorps F12CH présente une réactivité croisée avec le TfR de souris (figure 16-d), présent sur certaines cellules de l'hôte.

Le modèle animal utilisé pour tester ces anticorps reflète donc une partie des interactions possibles qui surviendraient lors d'une administration de l'anticorps F12CH chez l'homme.

Il est important de noter qu'au format utilisé, l'anticorps F12CH est dépourvu de fragment Fc et ne peut donc recruter les effecteurs du système immunitaire pour entraîner de réponse anti-tumorale de type ADCC (cytotoxicité cellulaire dépendante des anticorps) ou CDC (cytotoxicité dépendante du complément), ce qui montre que l'effet observé est propre au mode d'action de l'anticorps.

Ainsi, leur mode particulier de cytotoxicité, à savoir cytotoxicité indépendante des fonctions effectrices naturelles des anticorps de type ADCC ou CDC et associée à une augmentation du TfR à la surface des cellules cibles, rend les anticorps de l'invention particulièrement avantageux comme anticorps thérapeutiques à haut potentiel pour le traitement de cancers.

### Exemple IV : Effet des anticorps sur la prolifération de cellules sanguines mononuclées activées.

Les anticorps 3TF12 et 3GH7 au format bivalent sont testés pour les maladies auto-immunes ou encore pour les transplantations comme thérapie préventive contre le rejet de greffe.

L'expression du TfR/CD71 à la surface des cellules T jour un rôle essentiel dans l'activation et le maintien de la prolifération de lymphocytes T. Les anticorps inhibiteurs anti-TfR pourraient être utilisés comme immunosuppresseurs dans les maladies auto-immunes ou en prévention du rejet de greffe ou de la réaction du greffon contre l'hôte.

L'effet des anticorps 3TF12 et 3GH7 au format scFvCH (bivalent, 50 kD, produit en bactéries) a été testé sur la prolifération de cellules sanguines mononuclées humaines activées par la cytokine IL-2 (« PBMC »).

Des préparations effectuées dans les mêmes conditions d'anticorps 3TF12 (anti-TfR) (F12CH) et Bot (non spécifique) ont été testées (voir figure 17).

Une inhibition dose dépendante de la prolifération des PBMC activés est observée en présence d'anticorps anti-TfR F12.

A noter que les anticorps sont produits en bactérie et que des traces de lipolysaccharide (LPS) dans cette purification peuvent expliquer la stimulation de prolifération des PBMC en présence d'anticorps non spécifique Bot.

L'anticorps F12, au format dimérique scFvCH inhibe donc la prolifération IL-2 dépendante (50% d'inhibition à la concentration de 10 µg/mL) des lymphocytes périphériques et pourrait donc être utilisé comme immunosuppresseur.

### Exemple V : Production d'anticorps au format scFv-Fc et effet sur différentes lignées cellulaires.

Le format bivalent utilisé par les Inventeurs (scFv-CH) a permis d'améliorer significativement les effets antiprolifératifs des anticorps, initialement dans un format monovalent 3TF12 et 3GH7. Cependant, même si ce nouveau format possède un poids moléculaire plus élevé que le format standard scFv (55 versus 28 kD, supérieur à la limite de filtration rénale), sa demi-vie dans le sérum n'est que de quelques heures bien inférieure à celle d'anticorps complets d'isotype IgG (demi vie 21 à 24 jours chez l'homme pour les isotypes IgG1, 2 et 3). Ce poids moléculaire faible associé à l'absence de fragment Fc pourrait limiter considérablement la stabilité sérique et l'efficacité *in vivo* des anticorps (même si l'on observe un effet sur le modèle de xénogreffe chez la souris). De plus, la présence d'un fragment Fc permettrait en plus de la liaison au TfR, d'une part le recrutement du premier composant de la cascade du complément, le C1q et l'initiation de la cascade du complément, et d'autre part la liaison aux récepteurs Fc sur les cellules « natural killer » (NK) et les macrophages pour médier l'ADCC fonctions effectrices. Ceci pourrait, par un mécanisme indépendant de la déplétion en fer, augmenter l'activité cytotoxique de l'anticorps sur les cellules exprimant de forts taux de TfR à leur surface.

Les anticorps H7 et F12 sont préparés au format 110 kD scFv-Fc, ce qui signifie que l'on fusionne un fragment scFv à l'extrémité C- terminale les domaines constants CH2 et CH3 d'une IgG1 humaine. De telles protéines s'homodimérisent naturellement pour former une molécule bivalente de 110 kDa environ (2X 55 kDa).

Pour obtenir les anticorps au format scFv-Fc, les séquences VH et VL des scFv Bot, 3TF12 et 3GH7 sont sous-clonées dans le vecteur pFuse-hG1 (décrit dans Moutel,S.et al., 2009. A multi-Fc-species system for recombinant antibody production. BMC. Biotechnol. 9, 14) permettant d'obtenir les constructions pFuse-hG1-Bot-Fc, pFuse-hG1-F12-Fc et pFuse-hG1-H7-Fc. L'expression de ce format d'anticorps se fait par transfection de cellules CHO. Les cellules vont alors sécréter les scFv-Fc dans le milieu pendant 3 jours. Les anticorps sont ensuite purifiés sur résine de protéine A-agarose puis concentrés.

La figure 18 (a) illustre la production de l'anticorps F12 et Bot au format scFv-Fc. En conditions non-réductrices, on détecte le format dimérique des anticorps à 110 kD environ. En ajoutant du DTT les ponts disulfures sont réduits et les formes monomériques migrent à 55 kD comme attendu.

La figure 18 (b) montre que l'anticorps F12, au format scFv-Fc se lie sur les cellules HMC1.2 (lignée de mastocytome humain) et aux cellules murines BaF/3 (lignée pro-B hématopoïétique murine). Comme contrôle, on montre qu'il n'y a pas de liaison d'un anticorps dirigé contre la toxine botulique (Bot) aux cellules HMC1.2.

La figure 18 (c) montre que la prolifération des cellules HMC1.2 est inhibée par l'anticorps scFv-F12-Fc avec une plus forte intensité qu'avec le format scFv-F12 pour une même concentration. Les deux formats de l'anticorps non spécifique (Bot) n'ont aucun effet.

Le même résultat d'inhibition de croissance a été obtenu avec l'anticorps H7 (non montré ici).

La figure 19 montre que sur les cellules ERY-1, l'IC50 est inférieure à 0,1 µg/mL d'anticorps F12. Le format scFv-Fc est donc aussi efficace (voire plus) que le format bivalent scFvCH (figure 13-b).

L'anticorps F12 au format bivalent scFv-Fc a été testé sur la lignée UT-7, une lignée erythroleucémique humaine. Un fort effet inhibiteur (70% d'inhibition) de l'anticorps F12 est observé à la concentration de 10 µg/mL (figure 20).

### CONCLUSION GENERALE

Six anticorps différents spécifiques du récepteur de la transferrine reconnaissant des épitopes voisins sur le TfR et capables d'inhiber le ligand naturel du TfR, l'holotransferrine, ont été sélectionnés par présentation d'anticorps à la surface de phages pour leur capacité à être endocytés par des cellules cancéreuses vivantes, par endocytose médiée par récepteurs. Cette sélection fonctionnelle a permis d'obtenir six fragments d'anticorps monovalents anti-TfR humains (scFv anti-TfR), à savoir les fragments variable simple chaîne (scFv) d'un poids moléculaire d'environ 28 kD.

Les six fragments d'anticorps de type ligand fonctionnels scFv anti-TfR obtenus dans cette sélection interfèrent tous avec le ligand naturel holotransferrine (holo-Tf), qui se lie aux TfR, et perturbent donc tous la liaison de l'holo-Tf aux TfR.

Les fragments scFv anti-TfR qui présentent la plus grande affinité pour les TfR, à savoir 3TF12 (SEQ ID N°49) et 3GH7 (SEQ ID N°54), inhibent la prolifération de diverses lignées de cellules cancéreuses hématopoïétiques, et possèdent des propriétés anti-cancéreuses particulièrement intéressantes.

Le potentiel d'inhibition de la prolifération cellulaire des anticorps 3TF12 et 3GH7 a été amélioré en élaborant des formats d'anticorps bivalents de 55 kD présentant une affinité améliorée, à savoir F12CH et H7CH, du fait de la bivalence.

Ce changement de format (du monovalent au bivalent) a permis d'améliorer l'IC₅₀ relative à chaque anticorps.

Le mécanisme de cytotoxicité de ces anticorps endocytables à grande affinité est différent de celui des anticorps monoclonaux murins anti-TfR inhibiteurs de croissance décrits précédemment dans la littérature.

En effet, F12CH et H7CH induisent une augmentation du nombre de TfR au niveau de la surface des cellules (au lieu de le réduire en augmentant leur dégradation), tout en inhibant considérablement l'absorption cellulaire d'holo-Tf et en induisant la mort cellulaire par apoptose et/ou autophagie.

Ces propriétés ont permis de définir une nouvelle famille de fragments d'anticorps anti-TfR entièrement humains, appropriés pour l'immunothérapie des tumeurs qui dépendent du fer pour proliférer durablement et qui expriment un grand nombre de TfR.

Le fait que les anticorps de l'invention soient issus d'une banque d'anticorps humains est très avantageux puisqu'ils ne nécessitent donc pas d'étape d'humanisation pour leur transfert vers le développement préclinique.

Les six anticorps de l'invention (que ce soit dans leur format monovalent ou bivalent) sont susceptibles de cibler des épitopes légèrement différents (chaque épitope partageant des motifs avec le site de liaison du ligand naturel Tf) et présentent des affinités différentes pour leur cible.

Une utilisation alternative de l'un ou l'autre de ces anticorps en clinique pourrait s'avérer particulièrement avantageuse car elle permettrait d'éviter des phénomènes de résistance induite par le traitement.

Les anticorps de l'invention fonctionnent selon un mécanisme unique, encore jamais décrit à ce jour. Ils induisent la mort cellulaire des cellules cancéreuses en les privant en fer sans besoin de format multivalent agglutinant le TfR à la surface cellulaire.

Par ailleurs, une incubation de quelques jours, *in vitro,* en présence des anticorps entraîne une augmentation de TfR à la surface cellulaire. Si cette observation est reproduite *in vivo,* cette propriété unique augmentera la « visibilité » des cellules à cibler.

Le format anticorps utilisé *in vivo* est celui d'un scFv-dimérique bivalent, de 55 kDa environ.

Afin d'augmenter les propriétés anti-tumorales des anticorps de l'invention (aussi bien sous leur format monovalent que bivalent), on peut prévoir une modification du format de l'anticorps par ajout d'un fragment Fc pour obtenir un anticorps complet ou par pégylation, ce qui permettrait d'augmenter le temps de demi-vie des anticorps. Un format scFv-Fc (110 kDa) où le scFv est fusionné à la région Fc d'une immunoglobuline humaine de type IgG1 a été produit pour les anticorps scFv F12 et H7. Ce format reproduit les effets cytotoxiques des anticorps au format scFvCH *in vitro.* La présence de régions Fc pourrait, en outre, permettre le recrutement des effecteurs du système immunitaire et augmenter l'effet anti-tumoral de l'anticorps.

Les anticorps de l'invention (aussi bien sous leur format monovalent que bivalent), du fait de leur spécificité pour le TfR, de leur mode particulier de cytotoxicité et de leur faible immunogénicité liée à leur origine humaine, s'avèrent donc être des molécules thérapeutiques à haut potentiel pour le traitement du cancer.

## Revendications

1. Construction moléculaire **caractérisée en ce qu'**elle comprend
une séquence choisie parmi les séquences SEQ ID NO : 37, SEQ ID NO : 39, SEQ ID NO : 41, SEQ ID NO : 43, SEQ ID NO : 45, SEQ ID NO : 47 définissant chacune la totalité du domaine variable de la chaine lourde (VH) d'un anticorps ciblant le récepteur de la transferrine humaine (TfR), et
une séquence choisie parmi les séquences SEQ ID NO : 38, SEQ ID NO : 40, SEQ ID NO : 42, SEQ ID NO : 44, SEQ ID NO : 46, SEQ ID NO : 48 définissant chacune la totalité du domaine variable de la chaîne légère (VL) d'un anticorps ciblant le récepteur de la transferrine humaine (TfR).

2. Construction moléculaire selon la revendication 1, caractérisée en ce en ce que l'une au moins des séquences SEQ ID NO : 37 à SEQ ID NO : 48 est comprise dans l'une des séquences en acides aminés choisies parmi :
SEQ ID NO : 49, SEQ ID NO : 50, SEQ ID NO : 51, SEQ ID NO : 52, SEQ ID NO : 53, SEQ ID NO : 54,
chacune desdites séquences SEQ ID NO : 49 à SEQ ID NO : 54 comprenant le domaine variable de la chaîne lourde et de la chaîne légère d'un anticorps ciblant le récepteur de la transferrine humaine (TfR),
ladite séquence SEQ ID NO : 49 comprenant les séquences SEQ ID NO : 37 et SEQ ID NO : 38 reliées entre elles par le peptide de liaison présentant une séquence en acides aminés SEQ ID NO : 109,
ladite séquence SEQ ID NO : 50 comprenant les séquences SEQ ID NO : 39 et SEQ ID NO : 40 reliées entre elles par le peptide de liaison SED ID NO : 109,
ladite séquence SEQ ID NO : 51 comprenant les séquences SEQ ID NO : 41 et SEQ ID NO : 42 reliées entre elles par le peptide de liaison SED ID NO : 109,
ladite séquence SEQ ID NO : 52 comprenant les séquences SEQ ID NO : 43 et SEQ ID NO : 44 reliées entre elles par le peptide de liaison SED ID NO : 109,
ladite séquence SEQ ID NO : 53 comprenant les séquences SEQ ID NO : 45 et SEQ ID NO : 46 reliées entre elles par le peptide de liaison SED ID NO : 109,
ladite séquence SEQ ID NO : 54 comprenant les séquences SEQ ID NO : 47 et SEQ ID NO : 48 reliées entre elles par le peptide de liaison SED ID NO : 109.

3. Construction moléculaire selon la revendication 2, **caractérisée en ce qu'**elle se présente sous la forme d'un monomère ou d'un dimère.

4. Construction moléculaire selon l'une quelconque des revendications 1 à 3, **caractérisée en ce qu'**elle comporte en outre un fragment Fc.

5. Construction moléculaire selon l'une quelconque des revendications 1 à 4, **caractérisée en ce qu'**il s'agit d'un anticorps ou d'un fragment d'anticorps.

6. Séquences nucléotidiques **caractérisées en ce qu'**elles codent respectivement
- pour les séquences en acides aminés SEQ ID NO : 49 à SEQ ID NO : 54 **en ce qu'**elles sont représentées respectivement par les séquences SEQ ID NO : 103 à SEQ ID NO : 108, ladite SEQ ID NO : 103 comprenant les séquences SEQ ID NO°: 91 et SEQ ID NO°: 92 reliées entre elles par une séquence nucléotidique SEQ ID NO : 110,
ladite SEQ ID NO : 104 comprenant les séquences SEQ ID NO°: 93 et SEQ ID NO°: 94 reliées entre elles par SEQ ID NO : 110,
ladite SEQ ID NO : 105 comprenant les séquences SEQ ID NO°: 95 et SEQ ID NO°: 96 reliées entre elles par SEQ ID NO : 110,
ladite SEQ ID NO : 106 comprenant les séquences SEQ ID NO°: 97 et SEQ ID NO°: 98 reliées entre elles par SEQ ID NO : 110,
ladite SEQ ID NO : 107 comprenant les séquences SEQ ID NO°: 99 et SEQ ID NO°: 100 reliées entre elles par SEQ ID NO : 110,
ladite SEQ ID NO : 108 comprenant les séquences SEQ ID NO°: 101 et SEQ ID NO°: 102 reliées entre elles par SEQ ID NO : 110.

7. Molécule d'acide nucléique isolée **caractérisée en ce qu'**elle comprend au moins une des séquences nucléotidiques SEQ ID NO : 103 à SEQ ID NO : 108 telles que définies dans la revendication 6.

8. Vecteur d'expression **caractérisé en ce qu'**il comprend une molécule d'acide nucléique telle que définie à la revendication 7.

9. Cellule hôte isolée **caractérisé en ce qu'**elle comprend un vecteur d'expression tel que défini à la revendication 8.

10. Composition pharmaceutique comprenant une quantité thérapeutiquement efficace d'au moins une construction moléculaire telle que définie selon l'une quelconque des revendications 1 à 5 en association avec un véhicule pharmaceutiquement acceptable.

11. Composition selon la revendication 10, dans laquelle la construction moléculaire est utilisée pour vectoriser une (ou plusieurs) molécule(s) biologiquement active(s).

12. Composition selon la revendication 11, dans laquelle la construction moléculaire est utilisée pour le ciblage de liposomes et/ou de nanoparticules chargés d'un (ou plusieurs) agent(s) cytotoxique(s) et/ou d'un (ou plusieurs) agent(s) à visée diagnostique.

13. Composition selon l'une quelconque des revendications 10 à 12 pour son utilisation comme médicament dans le traitement de pathologies présentant une sur-expression du TfR, comme le cancer ou une pathologie auto-immune.

14. Composition selon la revendication 13, dans laquelle le cancer est du type lymphome ou leucémie.

## Patentansprüche

1. Molekulares Konstrukt, **dadurch gekennzeichnet, dass** es Folgendes umfasst:
eine Sequenz, die ausgewählt ist aus den Sequenzen mit den SEQ ID NO: 37, SEQ ID NO: 39, SEQ ID NO: 41, SEQ ID NO: 43, SEQ ID NO: 45, SEQ ID NO: 47, die jede die gesamte variable Domäne der schweren Kette (VH) eines Antikörpers bilden, der auf den Rezeptor des menschlichen Transferrin (TfR) abzielt, und
eine Sequenz, die ausgewählt ist aus den Sequenzen mit den SEQ ID NO: 38, SEQ ID NO: 40, SEQ ID NO: 42, SEQ ID NO: 44, SEQ ID NO: 46, SEQ ID NO: 48, die jede die gesamte variable Domäne der leichten Kette (VL) eines Antikörpers bilden, der auf den Rezeptor des menschlichen Transferrin (TfR) abzielt.

2. Molekulares Konstrukt, nach Anspruch 1, **dadurch gekennzeichnet, dass** mindestens eine der Sequenzen mit den SEQ ID NO: 37 bis SEQ ID NO: 48 in einer der Aminosäuresequenzen inbegriffen ist, die ausgewählt sind aus:
SEQ ID NO: 49, SEQ ID NO: 50, SEQ ID NO: 51, SEQ ID NO: 52, SEQ ID NO: 53, SEQ ID NO: 54,
wobei jede der Sequenzen mit den SEQ ID NO: 49 bis SEQ ID NO: 54 die variable Domäne der schweren Kette und der leichten Kette eines Antikörpers umfasst, der auf den Rezeptor des menschlichen Transferrin (TfR) abzielt,
wobei die Sequenz mit der SEQ ID NO: 49 die Sequenzen mit den SEQ ID NO: 37 und SEQ ID NO: 38 umfasst, die durch das Verbindungspeptid, das eine Aminosäuresequenz mit der SEQ ID NO: 109 darstellt, miteinander verbunden sind,
wobei die Sequenz mit der SEQ ID NO: 50 die Sequenzen mit den SEQ ID NO: 39 und SEQ ID NO: 40 umfasst, die durch das Verbindungspeptid mit der SEQ ID NO: 109 miteinander verbunden sind,
wobei die Sequenz mit der SEQ ID NO: 51 die Sequenzen mit den SEQ ID NO: 41 und SEQ ID NO: 42 umfasst, die durch das Verbindungspeptid mit der SEQ ID NO: 109 miteinander verbunden sind,
wobei die Sequenz mit der SEQ ID NO: 52 die Sequenzen mit den SEQ ID NO: 43 und SEQ ID NO: 44 umfasst, die durch das Verbindungspeptid mit der SEQ ID NO: 109 miteinander verbunden sind,
wobei die Sequenz mit der SEQ ID NO: 53 die Sequenzen mit den SEQ ID NO: 45 und SEQ ID NO: 46 umfasst, die durch das Verbindungspeptid mit der SEQ ID NO: 109 miteinander verbunden sind,
wobei die Sequenz mit der SEQ ID NO: 54 die Sequenzen mit den SEQ ID NO: 47 und SEQ ID NO: 48 umfasst, die durch das Verbindungspeptid mit der SEQ ID NO: 109 miteinander verbunden sind.

3. Molekulares Konstrukt, nach Anspruch 2, **dadurch gekennzeichnet, dass** es die Form eines Monomers oder eines Dimers aufweist.

4. Molekulares Konstrukt, nach einem der Ansprüche 1 bis 3, **dadurch gekennzeichnet, dass** es ferner ein Fragment Fc aufweist.

5. Molekulares Konstrukt, nach einem der Ansprüche 1 bis 4, **dadurch gekennzeichnet, dass** es sich um einen Antikörper oder ein Antikörperfragment handelt.

6. Nukleotidsequenzen, **dadurch gekennzeichnet, dass** sie jeweils kodieren
- für die Aminosäuresequenzen mit den SEQ ID NO: 49 bis SEQ ID NO: 54, dass sie jeweils durch die Sequenzen mit den SEQ ID NO: 103 bis SEQ ID NO: 108 dargestellt werden,
wobei die Sequenz mit der SEQ ID NO: 103 die Sequenzen mit den SEQ ID NO: 91 und SEQ ID NO: 92 umfasst, die durch eine Nukleotidsequenz mit der SEQ ID NO: 110 darstellt, verbunden sind,
wobei die SEQ ID NO: 104 die Sequenzen mit den SEQ ID NO: 93 und SEQ ID NO: 94 umfasst, die durch die SEQ ID NO: 110 miteinander verbunden sind,
wobei die SEQ ID NO: 105 die Sequenzen mit den SEQ ID NO: 95 und SEQ ID NO: 96 umfasst, die durch die SEQ ID NO: 110 miteinander verbunden sind,
wobei die SEQ ID NO: 106 die Sequenzen mit den SEQ ID NO: 97 und SEQ ID NO: 98 umfasst, die durch die SEQ ID NO: 110 miteinander verbunden sind,
wobei die SEQ ID NO: 107 die Sequenzen mit den SEQ ID NO: 99 und SEQ ID NO: 100 umfasst, die durch die SEQ ID NO: 110 miteinander verbunden sind,
wobei die SEQ ID NO: 108 die Sequenzen mit den SEQ ID NO: 101 und SEQ ID NO: 102 umfasst, die durch die SEQ ID NO: 110 miteinander verbunden sind.

7. Isoliertes Nukleinsäuremolekül, **dadurch gekennzeichnet, dass** es mindestens eine der Nukleotidsequenzen mit den SEQ ID NO: 103 bis SEQ ID NO: 108 nach Anspruch 6 umfasst.

8. Expressionsvektor, **dadurch gekennzeichnet, dass** er ein Nukleinsäuremolekül nach Anspruch 7 umfasst.

9. Isolierte Wirtszelle, **dadurch gekennzeichnet, dass** sie einen Expressionsvektor nach Anspruch 8 umfasst.

10. Pharmazeutische Zusammensetzung, die eine therapeutisch wirksame Menge mindestens eines molekularen Konstrukts nach einem der Ansprüche 1 bis 5 in Verbindung mit einem pharmazeutisch verträglichen Träger umfasst.

11. Zusammensetzung nach Anspruch 10, wobei das molekulare Konstrukt verwendet wird, um ein (oder mehrere) biologisch aktives (aktive) Molekül(e) zu vektorisieren.

12. Zusammensetzung nach Anspruch 11, wobei das molekulare Konstrukt verwendet wird zum Abzielen auf Liposome und/oder Nanopartikel, die mit einem (oder mehreren) zytotoxischen Wirkstoff(en) und/oder einem (oder mehreren) Wirkstoff(en) geladen sind, für Diagnosezwecke.

13. Zusammensetzung nach einem der Ansprüche 10 bis 12 für ihre Verwendung als Medikament bei der Behandlung von Pathologien, die eine Überexpression von TfR aufweisen, wie Krebs oder eine Autoimmunerkrankung.

14. Zusammensetzung nach Anspruch 13, wobei der Krebs des Lymphom- oder Leukämietyps ist.

## Claims

1. Molecular construction **characterised in that** it comprises a sequence chosen from the sequences SEQ ID NO: 37, SEQ ID NO: 39, SEQ ID NO: 41, SEQ ID NO: 43, SEQ ID NO: 45, SEQ ID NO: 47 which each define the total variable domain of heavy chain (VH) of an antibody targeting the human transferrin receptor (TfR), and a sequence chosen from the sequences SEQ ID NO: 38, SEQ ID NO: 40, SEQ ID NO: 42, SEQ ID NO: 44, SEQ ID NO: 46, SEQ ID NO: 48 which each define the total variable domain of light chain (VL) of an antibody targeting the human transferrin receptor (TfR).

2. Molecular construction according to claim 1, **characterised in that** at least one of sequences SEQ ID NO: 37 to SEQ ID NO: 48 is comprised in one of amino acid sequences chosen from:
SEQ ID NO: 49, SEQ ID NO: 50, SEQ ID NO: 51, SEQ ID NO: 52, SEQ ID NO: 53, SEQ ID NO: 54,
each of these sequences SEQ ID NO: 49 to SEQ ID NO: 54 comprising the variable domain of a heavy chain and of light chain of an antibody targeting the human transferrin receptor (TfR),
said sequence SEQ ID NO: 49 comprising the sequences SEQ ID NO: 37 and SEQ ID NO: 38 linked between them by a linking peptide having an amino acid sequence of SEQ ID NO: 109,
said sequence SEQ ID NO: 50 comprising the sequences SEQ ID NO: 39 and SEQ ID NO: 40 linked between them by a linking peptide having an amino acid sequence of SEQ ID NO: 109,
said sequence SEQ ID NO: 51 comprising the sequences SEQ ID NO: 41 and SEQ ID NO: 42 linked between them by a linking peptide having an amino acid sequence of SEQ ID NO: 109,
said sequence SEQ ID NO: 52 comprising the sequences SEQ ID NO: 43 and SEQ ID NO: 44 linked between them by a linking peptide having an amino acid sequence of SEQ ID NO: 109,
said sequence SEQ ID NO: 53 comprising the sequences SEQ ID NO: 45 and SEQ ID NO: 46 linked between them by a linking peptide having an amino acid sequence of SEQ ID NO: 109,
said sequence SEQ ID NO: 54 comprising the sequences SEQ ID NO: 47 and SEQ ID NO: 48 linked between them by a linking peptide having an amino acid sequence of SEQ ID NO: 109.

3. Molecular construction according to claim 2, **characterised in that** it is in the form of a monomer or a dimer.

4. Molecular construction according to any one of claims 1 to 3, **characterised in that** it contains in addition a Fc fragment.

5. Molecular construction according to any one of claims 1 to 4, **characterised in that** it is an antibody or a fragment of antibody.

6. Nucleotide sequences **characterised in that** they respectively code
- for amino acid sequences SEQ ID NO: 49 to SEQ ID NO: 54, **in that** they are respectively represented by the sequences SEQ ID NO: 103 to SEQ ID NO: 108,
said SEQ ID NO: 103 comprising the sequence SEQ ID NO: 91 and SEQ ID NO: 92 linked between them by a nucleic sequence SEQ ID NO: 110,
said SEQ ID NO: 104 comprising the sequence SEQ ID NO: 93 and SEQ ID NO: 94 linked between them by SEQ ID NO: 110,
said SEQ ID NO: 105 comprising the sequence SEQ ID NO: 95 and SEQ ID NO: 96 linked between them by SEQ ID NO: 110,
said SEQ ID NO: 106 comprising the sequence SEQ ID NO: 97 and SEQ ID NO: 98 linked between them by SEQ ID NO: 110,
said SEQ ID NO: 107 comprising the sequence SEQ ID NO: 99 and SEQ ID NO: 100 linked between them by SEQ ID NO: 110,
said SEQ ID NO: 108 comprising the sequence SEQ ID NO: 101 and SEQ ID NO: 102 linked between them by SEQ ID NO: 110.

7. Isolated nucleic acid molecule **characterised in that** it comprises at least one of the nucleotide sequences SEQ ID NO: 103 to SEQ ID NO: 108, such as that defined in claim 6.

8. Expression vector **characterised in that** it comprises a nucleic acid molecule such as that defined in claim 7.

9. Isolated host cell **characterised in that** they comprise an expression vector such as that defined in claim 8.

10. Pharmaceutical composition comprising a therapeutically efficient quantity of at least a molecular construction such as that defined according to any one of claims 1 to 5 in association with a pharmaceutically acceptable vehicle.

11. Composition according to claim 10, in which the molecular construction is used for vectorization of one or several biologically active molecule(s).

12. Composition according to claim 11, in which a molecular construction is used for the screening of liposomes and/or of nanoparticles bearing one or several cytotoxic agent(s) and/or one or several diagnostic purpose agent(s).

13. Composition according to any one of claims 10 to 12 for its use as drug in the treatment of pathologies having an over-expression of TfR, such as the cancer or an auto-immune disease.

14. Composition according to claim 13, in which the cancer is lymphoma or leukaemia.
